(19) **Europäisches Patentamt**

**European Patent Office**

**Office européen des brevets**

(11) Publication number: **0 212 489 B1**

(12) **EUROPEAN PATENT SPECIFICATION**

(45) Date of publication of patent specification: **30.11.94**

(51) Int. Cl.[5]: **C07K 13/00**, C12P 21/02, C07K 15/00, G01N 33/53, G01N 33/68, C12Q 1/00, A61K 39/395, A01K 67/00, A61K 37/02, A61K 39/00, //(C12P21/02,C12R1:91)

(21) Application number: **86110967.6**

(22) Date of filing: **08.08.86**

Divisional application 94102240.2 filed on 08/08/86.

(54) Anabolic activity modulator and uses thereof.

(30) Priority: **16.08.85 US 766852**

(43) Date of publication of application:
**04.03.87 Bulletin 87/10**

(45) Publication of the grant of the patent:
**30.11.94 Bulletin 94/48**

(84) Designated Contracting States:
**AT BE CH DE FR GB IT LI LU NL SE**

(56) References cited:
**WO-A-86/06280**
**US-A- 414 098**

**INTERNATL. JOURNAL OF CANCER, vol. 36, 1985; pp. 395-400&NUM;**

**NUCLEIC ACIDS RESEARCH, vol. 13, no. 12, June 1985, Eynsham, Oxford (GB); L. FRANSEN et al., pp. 4417-4429&NUM;**

(73) Proprietor: **THE ROCKEFELLER UNIVERSITY**
**1230 York Avenue**
**New York, NY 10021 (US)**

(72) Inventor: **Cerami, Anthony**
**76 Flanders-Drakestown Road**
**Flanders, NJ 07836 (US)**
Inventor: **Kawakami, Masanobu**
**1-35-20-205 Nishigahara**
**Kita-ku Tokyo 114 (JP)**

(74) Representative: **VOSSIUS & PARTNER**
**Postfach 86 07 67**
**D-81634 München (DE)**

Rank Xerox (UK) Business Services
(3.10/3.09/3.3.3)

PARASITE IMMUNOLOGY, no. 6, 1984; P.J. HOTEZ et al., pp. 203-209&NUM;

PROCEEDINGS OF THE NATL. ACADEMY OF SCIENCES USA, vol. 79, February 1982; M. KAWAKAMI et al., pp. 912-916&NUM;

PROCEEDINGS OF THE NATL. ACADEMY OF SCIENCES USA, vol. 80, May 1983; P.H. PEKALA et al., pp. 2743-2747&NUM;

NATURE, vol. 316 , 08 August 1985; B. BEUTLER et al., pp. 552-554&NUM;

JOURNAL OF IMMUNOLOGY, vol. 134, no. 3, March 1985; J.R. MAHONEY et al., pp. 1673-1675&NUM;

SCIENCE, vol. 229, 30 August 1985; B. BEUTLER et al., pp. 869-871&NUM;

NATURE, vol. 320, 17 April 1986; B. BEUTLER et al., pp. 584-588&NUM;

JOURNAL OF EXPERIMENTAL MEDICINE, vol. 161, May 1985, The Rockefeller University Press (US); B. BEUTLER et al., pp. 984-995&NUM;

IMMUNOLOGY LETTERS, vol. 11, 1985, Elsevier Science Publ. B.V.; A. CERAMI et al., pp. 173-177&NUM;

## Description

The present invention relates to pharmaceutical compositions for the treatment of cachexia and shock in humans, comprising an anti-cachectin (i.e. anti-modulator material) antibody.

The Applicants are authors or co-authors of several articles directed to the subject matter of the present invention. These articles are (1) Beutler et al., "Purification of Cachectin, a Lipoprotein Lipase-Suppressing Hormone Secreted By Endotoxin-Induced RAW 264.7 Cells", J. EXP. MED. 161 at 984-995 (May, 1985); (2) Beutler et al., "Lipopolysaccharide-Treated RAW 264.7 Cells Produce a Mediator Which Inhibits Lipoprotein Lipase in 3T3-L1 Cells", J. IMMUNOL. 134 (3) at 1673-1675 (March, 1985); and (3) Hotez et al., "Lipoprotein Lipase Suppression in 3T3-L1 Cells by a Haematoprotozoan-Induced Mediator From Peritoneal Exudate Cells." PARASITE IMMUNOL. (Oxf.) 6:203 (1984).

The research leading to the present invention was funded in part by grants from the National Institute of Health and the Rockefeller Foundation.

The present invention is directed to pharmaceutical compositions for the treatment of the effects of invasive stimuli such as infection upon animal hosts, and in particular, discloses the identification of materials which may participate in the host response to such invasive stimuli.

Several common physiological and biochemical derangements have been observed in various mammalian hosts responding to a variety of invasive stimuli such as bacterial, viral and protozoan infections, as well as tumors and endotoxemia. For example, these responses include fever, leukocytosis, hyperlipidemia, reduced food intake (cachexia) and activity, and other modifications in muscle, white blood cell and liver metabolism. In particular, recent studies aimed at elucidating the biochemical mechanism of cachexia in rabbits infected with Trypanosoma brucei noted that animals with a minimal parasite burden became moribund and exhibited an extreme hypertriglyceridemia, with a marked elevation of plasma very low density lipoprotein (VLDL); see C.A. Rouser and A. Cerami, MOL. BIOCHEM. PARASITOL. 1:31-38 (1980). The hypertriglyceridemic state was remarkable in view of the severe wasting diathesis that accompanied this experimental infection. The elevation of plasma VLDL was shown to result from a clearing defect, caused by a loss of peripheral tissue lipoprotein lipase (LPL) activity.

LPL activity has been observed by others, and it has been noted that this condition has existed when the human body was in shock; see E.B. Man, et al , "The Lipids of Serum and Liver in Patients with Hepatic Diseases", CLIN. INVEST. 24 at 623, et seq. (1945); see also John I. Gall in, et al., "Serum Lipids in Infection", N. ENGL. J. MED. 281 at 1081-1086 (November 13, 1969); D. Farstchi, et al., "Effects of Three Bacterial Infections on Serum Lipids of Rabbits", J. BACTERIOL. 95 at 1615, et seq. (1968); S.E. Grossberg, et al., "Hyperlipaemia Following Viral Infection in the Chicken Embryo: A New Syndrome", NATURE (London) 208 at 954, et seq. (1965); Robert L. Hirsch, et al., "Hyperlipidemia, Fatty Liver and Bromsulfophthalein Retention in Rabbits Injected Intravenously with Bacterial Endotoxin", J. LIPID. RES. 5 at 563-568 (1964); and Osamu Sakaguchi, et al., "Alternations of Lipid Metabolism in Mice Injected With Endotoxins", MICROBIOL. IMMUNOL. 23 (2) AT 71-85 (1979); R.F. Kampschmidt, "The Activity of Partially Purified Leukocytic Endogenous Mediator in Endotoxin Resistant C3H/HeJ Mice", J. LAB. CLIN. MED. 95 at 616, et seq. (1980); and Ralph F. Kampschmidt, "Leukocytic Endogenous Mediator", J. RET. SOC. 23 (4) at 287-297 (1978).

Additionally, publications are known by the Applicants that discuss the indentification and existence of "mediators" that appear to be involved in the host response to infection; and in particular, the following articles are listed: Sipe, J.D., et al., J. EXP. MED., 150:597-606 (1979); and Barney, C.C., et al., LIPTON, J.M. (Ed.), FEVER: INTERNATIONAL SYMPOSIUM, Dallas, Texas, April 11-12, 1979 XII + 263P. Raven Press: New York, Illus. ISBN 0-89004-451-1 (08877), 0 (0), pp.111-122 (1980); and Dinarello, C.A., "Human Leukocytic Pyrogen: Purification and Development of a Radioimmunoassay", PROC. NATL. ACAD. SCI. USA, 74(10) at 4624-4627 (October, 1977). All of the factors identified and investigated by each of the authors in the above noted articles and the articles authored or co-authored by Kampschmidt have been determined to comprise a single grouping of factors which has been identified as Interleukin 1. This determination has been documented in an article by Charles A. Dinarello, published in REVIEWS OF INFECTIOUS DISEASES, at Volume 6, No. 1 (January-February, 1984).

A similar deficiency of LPL activity was noted by Applicants in C3H/HeN mice after administration of Escherichia coli lipopolysaccharide (LPS). In contrast, the loss of LPL activity was not demonstrable in C3H/HeJ mice, which are genetically resistant to LPS. This resistance to endotoxin-induced LPL deficiency could be circumvented by the administration of serum obtained from endotoxin-sensitive animals that had been injected with LPS two hours previously. Similarly, resistance could be overcome by injecting conditioned medium from endotoxin-stimulated thioglycollate-elicited peritoneal macrophages, obtained from sensitive mice. These findings were set forth in full detail in copending Parent Application Publication No. US-A-4603106.

The above work was prompted by the Applicants' belief that "mediator" or "mediators" existed and were suspected to have a significant effect upon general anabolic activity of energy storage cells in the animal host. It was suspected that such "mediator" exerted a depressive effect upon the activity of certain anabolic enzymes, whose reduced activity was observed in instances where the host enter the condition known as shock, in response to infectious invasion. Resultingly, the relationship of the mediator produced by endotoxin-stimulated peritoneal mouse exudate cells, upon endotoxin-sensitive and endotoxin-insensitive mice alike, and the development through such investigation of a reagent for the measurement of anabolic enzyme activity was set forth in application Publication No. WO 83/00930.

Further investigation of this system was made in conjunction with the 3T3-L1 "Preadipocyte" model system, and the corresponding development of methods and associated materials for the development of antibodies to the "mediator" and other diagnostic procedures was set forth in application Publication No. WO 83/00930.

Thereafter, in application Publication No. US-A-4603106 the Applicants also established that the mediator substance that they had derived from the endotoxin stimulation of macrophage cells exhibited the activities of supressing the anabolic enzymes lipoprotein lipase, acetyl Coenzyme A Carboxylase and fatty acid synthetase, and further, inhibited the growth and differentiation of erythroid-committed cells.

Additional work performed since the filing of the afore mentioned patent application, as set forth in the above article (1) by Beutler et al., has resulted in the discovery that the earlier identified mediator substance contains within it a protein component which upon isolation and examination, appears to have a number of activities, which distinguishes it from both the mediator substance and the other factors identified in the art and known as Interleukin 1 and Interleukin 2. Accordingly, the present invention relates to the newly discovered protein isolate and its activities, and the application of corresponding antibodies in pharmaceutical compositions.

In accordance with the first aspect of the present invention, the modulator material that is the newly discovered isolate of the mediator substance is disclosed, and comprises a protein that exhibits the ability to substantially completely suppress the activity of the anabolic enzyme lipoprotein lipase (LPL), and likewise is capable of preventing the differentiation of fat cells and increasing the up take of glucose in muscle cells. This protein however, demonstrably lacks leucocyte activator activity, and in this latter characteristic distinguishes the known factors identified collectively as Interleukin 1. Of its affirmative activities, the present modulator material most significantly supresses LPL activity in distinction of Interleukin 2, and as such is believed to play a role in the onset of cachexia. A particular protein having the above properties has been isolated and found to possess a molecular weight of approximately 17 kilodaltons and a pI of approximately 4.8.

As mentioned earlier, the present modulator material may be prepared by the stimulation of macrophage cells with a material that accompanies an invasive stimulus. In particular, a sample of macrophage cells which may be derived from a variety of sources may be incubated with a stimulator material such as endotoxin or trypanosomes, to produce the mediator substance disclosed in copending US-A-4603106 Such incubation may take place for a period of time of up to twenty hours, and exact time limits will vary with the particular cells selected for incubation. Following such incubation, the medium may be appropriately treated as by centrifuging, to remove a supernatant containing the crude mediator substance. The mediator substance may then be precipitated from this supernatant with, for example, a 40-60% solution of ammonium sulfate. Thereafter, the crude mediator substance may be subjected to a series of known isolation techniques such as isoelectric focusing and gel electrophoresis, whereupon the modulator material will be recovered. The present invention naturally contemplates alternate means for preparation of the modulator material, including where applicable known genetic replicative techniques, and the invention is accordingly intended to cover such synthetic preparations within its scope.

More particularly, the modulator material disclosed comprises a polypeptide which upon investigation and examination appears to bear some similarities to the known protein identified as tumor necrosis factor (TNF) as found in humans.

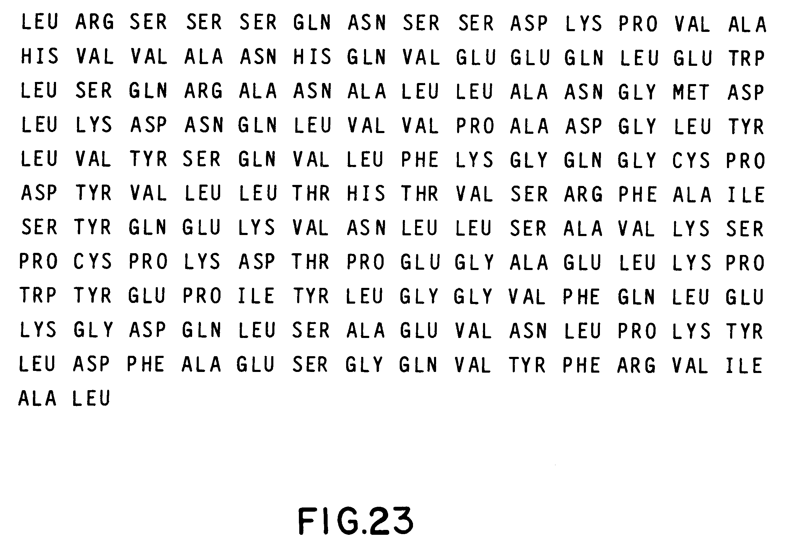

Accordingly, the present invention also discloses a protein having the above noted activities and characteristics, that displays the amino acid sequence set forth below and in Figure 23, as determined in mice.

4

```
LEU ARG SER SER SER GLN ASN SER SER ASP LYS PRO VAL ALA
HIS VAL VAL ALA ASN HIS GLN VAL GLU GLU GLN LEU GLU TRP
LEU SER GLN ARG ALA ASN ALA LEU LEU ALA ASN GLY MET ASP
LEU LYS ASP ASN GLN LEU VAL VAL PRO ALA ASP GLY LEU TYR
LEU VAL TYR SER GLN VAL LEU PHE LYS GLY GLN GLY CYS PRO
ASP TYR VAL LEU LEU THR HIS THR VAL SER ARG PHE ALA ILE
SER TYR GLN GLU LYS VAL ASN LEU LEU SER ALA VAL LYS SER
PRO CYS PRO LYS ASP THR PRO GLU GLY ALA GLU LEU LYS PRO
TRP TYR GLU PRO ILE TYR LEU GLY GLY VAL PHE GLN LEU GLU
LYS GLY ASP GLN LEU SER ALA GLU VAL ASN LEU PRO LYS TYR
LEU ASP PHE ALA GLU SER GLY GLN VAL TYR PHE ARG VAL ILE
ALA LEU
```

The foregoing sequence is both similar but in certain respects, distinctive from the known human TNF sequence, as will be set forth later on herein. Further, the recent isolation of the above amino acid sequence and the mRNA sequence facilitates reproduction of the modulator material by conventional recombinant genetic techniques.

In particular, the present invention relates to certain pharmaceutical compositions for the prevention and treatment of the occurrence of shock in mammals, comprising detecting the presence and shock promoting activity of the modulator material and thereafter administering an antibody to the modulator in an amount effective to prevent the development of shock. In another embodiment, the pharmaceutical compositions referred to herein are for the treatment of cachexia by the administration of antibodies to the modulator material, in view of the direct and significant effect that the modulator material has upon the activity of the anabolic enzyme LPL. These pharmaceutical compositions may contain said anti-cachectin antibody in combination with pharmaceutically acceptable carriers and/or other additives.

Other objects and advantages will become apparent to those skilled in the art from a review of the ensuing description which proceeds with reference to the following illustrative drawings.

FIGURE 1 is a graph depicting the results of bioassays of the modulator material taken from samples of fluids removed from an isoelectric focusing column.

FIGURE 2 is a graph depicting the results of bioassays of samples of the modulator material performed after gel electrophoresis, wherein slices of the gel were cut and bioactivity was then eluted from each slice by diffusion into ammonium bicarbonate solution. Units of bioactivity were plotted against the position at which each slice was made relative to the bottom of the gel.

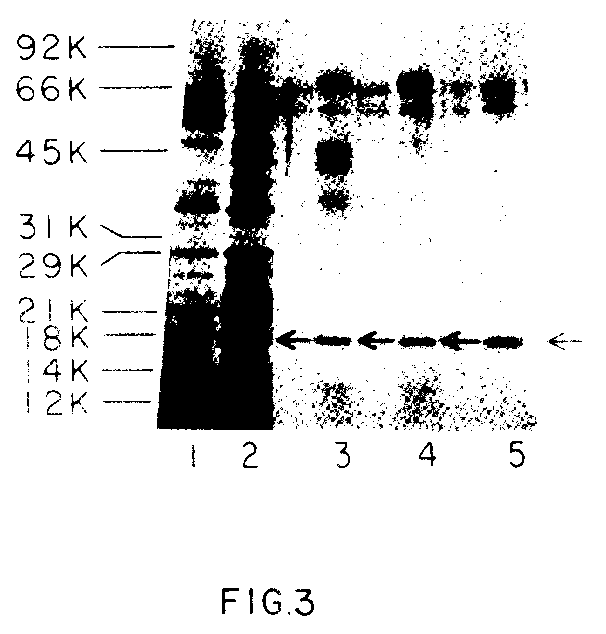

FIGURE 3 is an autoradiogram depicting the results of SDS-PAGE analysis of fractions from isolation of the modulator material. Samples of crude medium from endotoxin-induced and non-induced RAW 264.7 cell cultures, and purification intermediates were subjected to electrophoresis in a 10-15% linear polyacrylamide gradient gel. Samples were dialyzed against distilled water before electrophoresis. The following lengends identify the lanes shown in the autoradiogram. (1) 150 $\mu$l of non-induced RAW 264.7 cell medium; (2) 50 $\mu$l of induced RAW 264.7 cell medium; (3) 10 $\mu$l sample of pooled fractions from isoelectric focusing column; (4) 25 $\mu$l sample of Con A-Sepharose® filtrate; (5) 80-$\mu$l sample of the peak fraction obtained by PAGE under non-denaturing conditions (homogeneous cachectin). Arrows indicate cachectin, which is entirely absent from the medium of non-induced cells, but is a major constituent of the medium of induced cells. Cachectin is successively enriched by the purification steps listed above. The high molecular weight bands visible in all lanes (including spacer lanes) are $\beta$-mercaptoethanol contaminants, and are not present in the samples themselves.

FIGURE 4 is a graph depicting the results of SDS-PAGE analysis of the purified modulator material of the present invention. A 20-$\mu$g sample of purified cachectin was boiled for two minutes in the presence of SDS and subjected to electrophoresis under denaturing conditions in a 10-15% linear polyacrylamide gel. Serial 2-mm slices of this gel were electroeluted and the electroelution fractions were diluted 1:100 with DME containing 10% FBS, and 10-$\mu$l alequots were assayed for bioactivity.

FIGURES 5A and 5B are graphs depicting the results of radiolabelled binding studies conducted with the purified modulator material.

FIGURES 6A and 6B are graphs depicting the results of Scatchard analyses of the radioiodination and binding studies performed with the modulator material of the present invention.

FIGURE 7 is an autoradiogram corresponding to the Mono QFPLC anion exchange) chromatography conducted with crude concentrated RAW 267.4 medium, with an inset photograph revealing a silver-stained SDS polyacrylamide gradient gel showing the separation of crude material and successive column fractions.

FIGURE 8 is an autoradiograph depicting Superose 12 (FPLC gel filtration) chromatography of fractions taken from the Mono-Q fractionation performed initially. An inset photograph is included showing the SDS polyacrylamide gradient gel showing the electrophoretic profile of successive fractions derived from this filtration.

FIGURE 9 is a graph depicting the results of the addition to 3T3-L1 cells of purified Il-1 and the present modulator material.

FIGURE 10 is a graph depicting the results of the addition of recombinant IL-1 and a goat neutralizing antiserum to cultured 3T3-L1 cells.

FIGURE 11 is a graph depicting the results of a radio receptor assay of binding of the modulator material, wherein, in segment A, recombinant IL-1 is allowed to compete with the labeled modulator, and in segment B unlabeled modulator material is allowed to compete in the same system. Binding is expressed in terms of the percentage of input CPM bound by 3T3-L1 cells in each assay. Brackets indicate average binding, ± SEM.

FIGURE 12 is a photograph illustrating the results of the isolation of mRNA from TA1 cells to which conditioned media from RAW 264.7 cells was added to preadipocyte cultures two days prior to confluence.

FIGURE 13 is a photograph of the results of dot-blot testing performed in similar fashion to FIGURE 12.

FIGURE 14 is a photograph depicting the results of dot-blot tests based upon transcription assays performed to determine whether the modulator inhibition of adipose-specific mRNA accumulation is transcriptionally related.

FIGURE 15A is a graph depicting the results of the addition of the modulator material to adipocyte cultures that were differentiated by the procedure relating to FIGURE 12. RNA was isolated at indicated times after exposure to the modulator material and was applied to nitrocellulose with a dot-blot apparatus after which the filters were probed with the indicated cDNAs, washed, autoradiographed and then scanned.

FIGURE 15B is a photograph representing the results of Northern analysis of RNA, where the samples were first electrophoresed in an agarose formaldehyde gel and after washing with distilled water, sodium phosphate and EDTA, the gels were transferred to nitrocellulose.

FIGURE 16 is a graph depicting the results of comparative food intake experiments, measuring actual food intake between control mice and mice that received quantities of the present modulator.

FIGURE 17 is a graph depicting the relative change in body mass of the animals that participated in the comparative food intake experiments depicted in FIGURE 16.

FIGURE 18 is a graph depicting the effects upon the animals' body mass in the instance where administration of the modulator was discontinued.

FIGURE 19 illustrates the results of immunoprecipitation of radiolabeled samples of the modulator material of the present invention.

FIGURE 20 is a photograph of the results of a Western blot analysis performed after electrophoresis, comparing immune and pre-immune sera.

FIGURE 21 is a graph of the results of endotoxin $LD_{50}$ in mice treated with immune and non-immune sera.

FIGURE 22 is a graph depicting a Kaplan-Meier survival plot following LPS treatment of BALB/c mice that were split into five groups and innoculated with intraperitoneal injection of anti-modulator serum either six or three hours before, concurrent with or three or six hours following injection of a quantity of E. coli.

FIGURE 23 is a depiction of the amino acid sequence of the modulator material, as recovered from mice.

The present invention discloses the isolation and identification of a particular factor hereinafter alternately referred to as the modulator material or cachectin, that has been found to be present in macrophages and macrophage cell lines that are stimulated by materials referred herein as stimulator materials, that characteristically accompany an invasive stimulus, such as bacteria, virus, certain tumors, protozoa and other toxins such as endotoxin. As with the mediator substance disclosed in Parent Application Publication No. US-A-4603106, the present modulator material, which has been determined to be a component of the former, appears capable of causing the metabolism of certain of the cells of a mammal to switch from an anabolic state to a catabolic state, for the apparent purpose of mobilizing the host against the invasive stimulus.

In particular, the present modulator material appears to supress the activity of the anabolic enzyme lipoprotein lipase (LPL), and thus suggests that it functions as part of a communication system between the immune system of the host and its energy storage tissue. The modulator material also appears to prevent the differentiation of fat cells, and increases the up take of glucose in muscle cells and is therefore theorized to operate in response to invasive stimuli to produce and exert an effect on energy storage tissue such as adipose tissue, muscle and the like, to meet the inpending need for energy to combat the invasion. The modulator material thus promotes a conversion of the cellular activity of the host to a catabolic state to facilitate the supply of such energy. If the invasion is of short duration, the host can quickly recover and replenish the expended energy; however, if the invasion is of a continuous or chronic nature, complete energy depletion, cachexia, shock and ultimately, death may result.

Accordingly, one form of the present modulator material has been confirmed to comprise a protein that possesses a molecular weight of approximately 17,000 daltons and an isoelectric point of approximately 4.7-4.8. This particular modulator material exists as a dimer or greater multimer, as revealed by the results of the investigations that led to its isolation, which are set forth in Examples I and II, below. These tests likewise confirm that the modulator material binds to specific high-affinity receptors on the cells with which it interacts. In particular, the data in Example I suggest the existence of approximately $10^4$ high-affinity binding sites per cell, and a corresponding association constant ($K_a$) of $3 \times 10^9$. As will be borne out later on herein, this in turn, suggests both a structural and functional distinction between the present modulator material and the known factors of the prior art, as the activity and in particular the binding mechanism of the present modulator material is clearly distinct. A further characteristic of the modulator material is that it is capable of raising antibodies to itself that are effective to prevent endotoxin-induced shock. This will be set forth in Example X.

In addition to the activities set forth above, the present modulator material is distinctive in those activities that it lacks. In particular, the modulator material distinguishes the mediator substance in its inability to supress either of the anabolic enzymes acetyl Coenzyme A carboxylase and fatty acid synthetase, or to prevent the growth and differentiation of erythroid-committed cells. In similar fashion, the modulator material distinguishes the known factor Interleukin 1 by its lack of leucocyte activator activity. Also, the modulator appears to lack the ability to cause fever in mice, or muscle protein degradation. The distinctions in activity that serve as identifying features of both the modulator material and its precursor mediator substance are set forth in greater detail in the Examples presented later on herein.

The preparation of the modulator material was discussed in brief earlier herein, and is confirmed to be capable of proceeding by the initiation of the incubation of a variety of cells with stimulator materials from invasive stimuli. In particular, the cell line RAW 264.7 may be utilized to initiate the production of the precursor mediator substance from which the modulator material may be isolated. In particular, the development of the murine macrophage cell line RAW 264.7 as discussed in greater detail hereinafter, facilitates the isolation of the modulator material in quantities large enough to permit analysis and purification to be conducted. Naturally, other cell lines or other sources for the development of either the precursor mediator substance from which the modulator material is thereafter isolated, or the modulator material itself, are contemplated herein and the present invention is accordingly not limited. Thus, alternate means such as by genetic replication are contemplated herein in accordance with the present invention.

A modulator material in accordance with the present invention was isolated and analyzed in mice as set forth in Example II herein, and found to bear some similarity to the known protein tumor necrosis factor (TNF) as found in humans. Further work performed after the completion of the experiments set forth in Example II, has resulted in the elucidation of the complete sequence of this modulator protein, and this sequence is presented herein in FIGURE 23. It has therefore been determined that the mature modulator protein sequence is defined by 156 amino acids. Moreover, the mRNA of the modulator associated with this amino acid sequence has also been identified and has been employed to prepare the recombinant form of this disclosed protein. The technique employed, comprised the isolation of the mRNA from endotoxin-induced RAW 264.7 cells, followed by the preparation of an appropriate vector containing the mRNA which was then introduced into E. coli. Transformation took place with high efficiency (eg. approximately $10^8$ recombinant bacteria per $\mu$g DNA) using the recombinant plasmid DNA. Thereafter, a synthetic oligonucleotide probe (45 np in length) was constructed and was used to screen the cDNA library by in situ hybridization to lysed bacterial colonies, and sequencing was performed by the dideoxynucleotide method.

The foregoing procedure is excerpted from unpublished experiments and can be seen to involve many of the general principles of recombinant technology that are well known in the art. Accordingly, the present invention contemplates the preparation of the present modulator material, and in particular the material having the amino acid sequence set forth in FIGURE 23, by known recombinant techniques.

While the amino acid sequence of the modulator as set forth in Example II appears to be similar to human TNF, differences in the numbers of particular amino acid units suggest that distinctions as well as similarities between the proteins exist. In particular, the study in Example II compared modulator material derived from the stimulation of mouse cells with known structural data recently generated regarding human tumor necrosis factor and found both similarities and differences, the latter primarily in the numbers and disposition of certain of the amino acid residues. The study also revealed that certain activity of mouse-developed modulator material is present that is suggestive of anti-neoplastic capabilities similar to those of human TNF.

In connection with the above, the modulator material may be utilized for the purpose of treating the effects of post infection, such as cachexia and shock. In particular, the modulator material may be used to produce antibodies to itself in a variety of mammals, by known techniques such as the hybridoma technique utilizing, for example, fused mouse spleen lymphocytes and myeloma cells. The resulting antibodies could then be prepared in a suitable pharmaceutical composition and administered to the host bearing the invasive stimulus, to avert or treat the condition of cachexia.

In similar fashion to the treatment of cachexia, the antibodies to the modulator material may be prepared and may be utilized to treat the condition of shock. The exact quantities, intervals of administration and administrative techniques respecting each of the pharmaceutical compositions may vary in accordance with those known in the medical arts, and upon the specific instruction of a qualified physician or veterinarian. The qualified physician or veterinarian will also consider factors such as age, weight, general health and concentration of the modulator material having bearing in such regard.

Antibody(ies) to the modulator material can be produced and isolated by standard methods including the well known hybridoma techniques. The antibody(ies) can be used in another species as though they were antigen(s) to raise antibody(ies) . Both types of antibody(ies) can be used to determine the presence and extent of modulator material activity. For convenience, the antibody(ies) to the modulator material will be referred to herein as $Ab_1$ and antibody(ies) raised in another species as $Ab_2$.

The presence of modulator material activity in hosts suspected of harboring an invasive stimulus can be ascertained by the usual immunological procedures applicable to such determinations. A number of useful procedures are known. Three such procedures which are especially useful utilize either the modulator material labeled with a detectable label, antibody $Ab_1$ labeled with a detectable label, or antibody $Ab_2$ labeled with a detectable label. The procedures may be summarized by the following equations wherein the asterisk indicates that the particle is labeled, and "Mod" stands for the modulator material:

A. $Mod^* + Ab_1 = Mod^*Ab_1$
B. $Mod + Ab_1^* = ModAb_1^*$
C. $Mod + Ab_1 + Ab2^* = ModAb_1 Ab_2^*$

The procedures and their application are all familiar to those skilled in the art and accordingly may be utilized within the scope of the present invention. The "competitive" procedure, Procedure A, is described in U.S. Patent Nos. 3,654,090 and 3,850,752. Procedure C, the "sandwich" procedure, is described in U.S. Patent Nos. RE 31,006 and 4,016,043. Still other procedures are known such as the "double antibody", or "DASP" procedure.

In each instance, the modulator material forms complexes with one or more antibody(ies) or binding partners and one member of the complex is labeled with a detectable label. The fact that a complex has formed and, if desired, the amount thereof, can be determined by known methods applicable to the detection of labels.

It will be seen from the above, that a characteristic property of $Ab_2$ is that it will react with $Ab_1$. This is because $Ab_1$ raised in one mammalian species has been used in another species as an antigen to raise the antibody $Ab_2$. For example. $Ab_2$ may be raised in goats using $Ab_1$ as an antigen. $Ab_2$ therefore would be anti-rabbit antibody raised in goats. For purposes of this description and claims, $Ab_1$ will be referred to as a modulator material antibody and $Ab_2$ will be referred to an an antibody reactive with a modulator material antibody or, in the alternative, an "anti-antibody".

The labels most commonly employed for these studies are radioactive elements, enzymes, chemicals which fluoresce when exposed to ultraviolet light, and others.

A number of fluorescent materials are known and can be utilized as labels. These include, for example, fluorescein, rhodamine and auramine. A particular detecting material is anti-rabbit antibody prepared in goats and conjugated with fluorescein through an isothiocyanate.

The modulator material or its binding partner(s) can also be labeled with a radioactive element or with an enzyme. The radioactive label can be detected by any of the currently available counting procedures. The preferred isotope may be selected from $^{14}C$, $^{131}I$, $^{3}H$, $^{125}I$ and $^{35}S$.

8

Enzyme labels are likewise useful, and can be detected by any of the presently utilized colorimetric, spectrophotometric, fluorospectrophotometric or gasometric techniques. The enzyme is conjugated to the selected particle by reaction with bridging molecules such as carbodiimides, diisocyanates, glutaraldehyde and the like. Many enzymes which can be used in these procedures are known and can be utilized. The preferred are peroxidase, $\beta$-glucuronidase, $\beta$-D-glucosidase, $\beta$-D-galactosidase, urease, glucose oxidase plus peroxidase and acid phosphatase. U.S. Patent Nos. 3,654,090; 3,850,752; and 4,016,043 are referred to by way of example for their disclosure of alternate labeling material and methods.

A particular assay system developed and utilized in accordance with the present invention, is known as a receptor as say. In a receptor assay, the material to be assayed is appropriately labeled and then certain cellular test colonies such as the 3T3-L1 cell system are first differentiated then innoculated with a quantity of the labeled material after which binding studies are conducted to determine the extent to which the labeled material binds to the cell receptors. In this way, differences in activity between materials can be ascertained. This procedure is specifically illustrated in Example 1 later on herein.

Thus, as described in detail in Example 1 a purified quantity of the modulator material was radiolabeled, after which binding studies were carried out using recently differentiated 3T3-L1 cells. A solution was prepared that contained varying quantities of labeled modulator material and cell samples were innoculated and thereafter incubated. The resulting cell monolayers were then washed, solubilized and then counted in a gamma counter for a length of time sufficient to yield a standard error of <5%. This data was then subjected to Scatchard analysis after which observations and conclusions regarding material activity were drawn. While the foregoing protocol is exemplary, it illustrates the manner in which a receptor assay may be performed and utilized, in the instance where the cellular binding ability of the assayed material may serve as a distinguishing characteristic.

As indicated earlier, the following examples set forth the details of the isolation and identification of the present modulator material, and the observations noted as to its activity, defining both the distinctions and similarities in activity between the present modulator material and those factors identified earlier both by applicants and by others in the field. Naturally, the specific materials and techniques set forth hereinafter are exemplary only and may vary, so that the following is presented as illustrative but not restrictive of the present invention.

## EXAMPLE 1

In the present example, the isolation of the present modulator material from 3T3-L1 cells is set forth. Investigation of the isolate reveals that it is a 17 kilodalton protein that binds to adipocytes and several other cell types by means of specific high-affinity receptors.

## MATERIALS AND METHODS

3T3-L1 Cell Culture and Bioassay. 3T3-L1 cells were plated in 24-well Linbro plates (Flow Laboratories, Inc., McLean, Virginia) at a density of 100,000 cells per well in Dulbecco's modified Eagle's medium (DME) (Gibco Laboratories, Grand Island, New York) supplemented with 10% calf serum, and maintained at 37°C in a 10% $CO_2$ environment. Medium was changed every other day; after one week, cells were differentiated by incubation for 48 hours in medium supplemented with 10% fetal bovine serum (FBS), 10 $\mu$g/ml bovine insulin, 0.5 mM methylisobutylxanthine, and 1.0 $\mu$M dexamethasone. Differentiated cells were maintained in medium containing 10% FBS and 50 ng/ml bovine insulin (changed every other day) until their use for bioassay or binding studies one week later.

Samples to be assayed, in volumes $\leq$ 0.1 ml, were applied to individual wells of differentiated 3T3-L1 cells, each containing 1.0 ml of medium, and placed in a 10% $CO_2$ incubator at 37°C for twelve to eighteen hours. At the end of this incubation, the medium was aspirated and replaced with 0.5 ml of DME containing 10% FBS, 50 ng/ml insulin, and 10 U/ml heparin. After one hour at 37°C, the heparin-releasable LPL activity was measured according to the method of Nilsson-Ehle and Schotz.

Percent of total LPL suppression (PTS) in a sample with LPL activity x was defined as follows: PTS = (C-x/C-m) x 100, where C is the LPL activity produced by wells of 3T3-L1 cells that have not been exposed to the crude mediator, and m is the LPL activity of heparinized medium alone. To avoid day-to-day assay variability, the unknown sample results were compared with a standard preparation of the crude mediator substance included in all bioassays. This standard preparation of crude mediator was aliquoted into small tubes and stored at -80°C. 1 U of bioactivity was defined as the amount of cachectin yielding a PTS equivalent to that achieved by addition of 1.0 $\mu$l of the standard to the 1.0-ml assay system. Use of a standard solution of the crude mediator also assured a linear estimate of the amount of bioactivity present in unknown samples.

Mediator Production. RAW 264.7 cells were grown to confluence in RPMI maintained at 37°C in a 5% $CO_2$ environment. Before induction of mediator secretion, RAW 264.7 cells were washed extensively with Hanks' balanced salt solution (Gibco Laboratories) buffered with 25 mM Hepes, pH 7.4 (Research Organics, Inc., Cleveland, Ohio) to remove contaminating serum proteins. Each flask was stimulated with 100 ml of RPMI 1640 medium containing 1 μg/ml of E. coli LPS (Difco Laboratories, Inc., Detroit, Michigan) and 50 mM Hepes, pH 7.4. After twenty-two hours of incubation at 37°C, the medium was filtered through a 0.22 μm filter and frozen at -20°C.

Purification of the Mediator. 855 ml of stored frozen medium was thawed and concentrated under 50 psi of nitrogen at 4°C using a stirred cell with a PM-10 filter (Amicon Corp., Danvers, Massachusetts). After concentration to 2% of the initial volume, the retained protein solution was repeatedly diluted with distilled water and reconcentrated to desalt the sample before isoelectric focusing.

Isoelectric focusing was performed at 4°C in a water-cooled column with a 400 ml capacity. A glycerol gradient was prepared in this column with the protein sample layered in the center as follows: a linear gradient of glycerol from 40 to 21% (vol/vol) was added; the sample was supplied with a peristaltic pump; and a glycerol gradient of 19 to 0% (vol/vol) was layered above the sample. Both the upper and lower segments of the gradient contained Servalyte®, pH 3-10 (Serva Fine Biochemicals, Inc., Garden City Park, New York), diluted 1:16 with water. The sample was prepared by mixing the salt-free concentrate (55 ml vol.) with 4.3 ml. Servalyte® pH 3-10, and 16.7 g glycerol. The anode solution (at the bottom of the column) was 0.01 M imidodiacetic acid in 40% glycerol, and the cathode solution (at the top of the column) was 0.01 M ethylenediamine in $H_2O$. The separation was carried out at an initial current of 18 mA and an initial voltage of 630 V. The voltage was increased as current declined over the course of the run, but power output was not allowed to exceed 16W.

After twenty four hours, the column was drained under gravity into 150-drop fractions. Precipitated proteins were removed by centrifugation at 3,000 g for twenty minutes at 4°C. A drop of phenol red solution was added to each decanted supernatant, and acidic samples were neutralized by addition of saturated tribasic sodium phosphate solution, while basic samples were neutralized by addition of 30% phosphoric acid solution. Cachectin bioactivity was determined by assaying 2 μl from each fraction, corresponding to the peak of bioactivity, which were pooled for further purification.

The pooled peak fractions were dialyzed at 4°C against several changes of Dulbecco's phosphate-buffered saline (PBS) (Gibco Laboratories) to remove ampholytes and glycerol. The dialyzed material (94 ml in volume) was passed over a concanavalin A (Con A) Sepharose® column (Pharmacia Fine Chemicals, Uppsala, Sweden) with a bed volume of 2.0 ml for one hour at 4°C. An additional 10 ml of PBS was then run through the resin and pooled with the rest of the filtrate. The filtrate was dialyzed against two changes of distilled water at 4°C and lyophilized.

The sample was redissolved in 2 ml of a solution containing 30 mM Tris-HCl buffer, pH 6.8, 5% glycerol, 1 mM β-mercaptoethanol, and 0.002% bromphenol blue, and then subjected to electrophores is under nondenaturing conditions. The sample was applied across the stacking gel of a 1.5 mm x 14.2 cm x 13.6 cm slab gel with a 6-13% linear polyacrylamide gradient. A Tris-glycine buffer system was used as follows: the cathode buffer was 38 mM Tris base and 40 mM glycine; the anode buffer was 63 mM Tris base and 50 mM HCl; the stacking gel buffer was 0.125 M Tris-HCl, pH 6.8; and the separating gel buffer was 0.375 M Tris-HCl, pH 8.8 (final concentrations). In addition to acrylamide (29.2%), the monomer solution contained 0.8% bis-acrylamide. To remove impurities, the monomer solution was stirred overnight with Amberlite® MB-1 resin (Mallinckrodt, Inc., St Louis, Missouri).

The sample was electrophoresed at 4°C with a constant current of 20 mA until the bromphenol blue marker reached the bottom of the gel. After completion of electrophoresis, 1 cm of gel was removed from the lateral margins of the slab, since voltage artifacts caused nonhorizontal migration in these regions, and the remainder of the gel was sliced horizontally at 2-mm intervals. The protein was recovered from each slide by allowing it to diffuse into 2 ml of a 50 mM ammonium bicarbonate solution in a polypropylene tube over a period of twelve hours at 4°C with gentle agitation. Electroelution was carried out in a tube gel apparatus against a 3,500-dalton-cutoff dialysis membrane, as described by Francis, R.T., Jr., et al., J. CHROMATOGR., 298:115(1984).

The fractions thus obtained were assayed for activity, and subjected to polyacrylamide gel electrophoresis in a denaturing system containing sodium dodecyl sulfate (SDS-PAGE) in 10-15% linear gradient gels under nonreducing and reducing conditions to assess purity. The protein bands were visualized by silver staining as previously described by Wray, E., et al., ANAL. BIOCHEM. 118:197(1981).

Analytical Column Chromatography. Gel filtration was carried out using fine grade Sephadex® G-75 (Pharmacia Fine Chemicals) in a 7 mm x 1 m glass column, equilibrated with 0.1 M ammonium bicarbonate, pH 7.8. Enzyme grade urea (Bethesda Research Laboratories, Bethesda, Maryland) was added to the sample and the eluant buffer in experiments aimed at determining cachectin subunit size, and fractions were individually dialyzed against PBS before bioassay.

Studies With Radioiodinated Cachectin. Purified cachectin was radiolabeled by the iodogen method. The free iodide was removed by Sephadex® G-25 gel filtration followed by extensive dialysis. Binding studies were carried out using 24-well Linbro plates containing recently differentiated 3T3-L1 cells ($4 \times 10^5$ cells per 0.2 ml system). The solution used for binding studies consisted of DME medium, containing 10% FBS, 50 ng/ml insulin, and 50 mM Hepes buffer, pH 7.4. Varying quantities of labeled cachectin were added to this solution. Except where otherwise noted, the incubations were carried out on ice for four hours, with slow agitation. The cell monolayers were then washed three times with 1.0 ml of medium, solubilized with 1.0 of 1 M NaOH, and counted in a gamma counter for a length of time sufficient to yield a standard error of <5%.

The muscle cell line C2 (the kind gift of Dr. Helen Blau of Stanford University Medical School, Palo Alto, California) was also analyzed for cachectin binding. Cells were grown to confluence in 24-well Linbro plates. The plates were pretreated with calf skin collagen (Calbiochem-Behring Corp., La Jolla, California) by autoclaving 50 mg of collagen in 35 ml water and applying the sterile solution to each of the 24 wells per plate. The solution was then aspirated and the plates allowed to dry under ultraviolet light in a laminar flow hood. Growth medium used was DME supplemented with 20% FBS and 0.5% chick embryo extract (Gibco Laboratories). Cultures were maintained at 37°C in a 5% $CO_2$ environment. The medium was changed daily, and cells were induced to form myotubules when they reached confluence by addition of DME containing 2% horse serum (Gibco Laboratories). After forty-eight hours, the cells were used for binding studies. Measurement of cachectin binding to C2 myotubules was performed in a manner identical to that described for 3T3-L1 cells.

NCS female mice were obtained from the Laboratory Animal Research Center of The Rockefeller University. Mouse erythrocytes (RBC) were obtained by filtration of heparinized mouse blood over a microcrystalline cellulose column as previously described. Splenic lymphocytes were obtained by Ficoll®-Hypaque (Pharmacia Fine Chemicals) sedimentation. Measurements of cachectin binding to erythrocytes and lymphocytes were undertaken with the use of the same procedures applied in the case of 3T3-L1 cells. However, incubation with labeled cachectin was performed in 1.5-ml Eppendorf® tubes (Brinkmann Instruments, Inc., Westbury, New York). $4 \times 10^7$ mouse RBC and $1 \times 10^7$ mouse lymphocytes were used in each binding assay. Washing was accomplished by centrifuging three times at 4°C using a microfuge (Beckman Instruments, Inc., Fullerton, California). The pellets of cells were counted directly.

Crude hepatocyte membranes were prepared by homogenization of 2 g mouse liver (wet weight) in 10 ml of 5 mM Hepes, pH 7.4. The homogenate was diluted to 50 ml with the same buffer and centrifuged at 200 g at 4°C for five minutes. The supernatant was divided into 2-ml aliquots and pelleted at 48,000 g in a ten minute spin. The resulting pellets were each suspended in 0.5 ml of DME supplemented with 10% FBS, to which labeled cachectin (1 ng/ml) was added, with or without unlabeled cachectin (300 ng/ml). Binding was allowed to occur for four hours at 4°C. The membranes were then washed three times with fresh medium by centrifugation at 48,000 g. The pellets were counted for radioactivity.

Assays of Interleukin 1 (IL-1) Activity. These were carried out as previously described, using recombinant IL-1 as a standard preparation.

Protein Determination. Estimates of protein concentration were made by means of a commercial Coomassie®-based protein reagent (Bio-Rad Laboratories, Richmond, California).

RESULTS

Preparation of Cachectin from RAW 264.7 Cells and Characteristics in Crude Solution. ~$10^5$ U of cachectin bioactivity in 50 ml of medium could be obtained from a confluent flask containing $10^8$ RAW 264.7 cells. The specific activity of the crude mediator was routinely observed to be ~$4 \times 10^5$ U/mg protein. Very little additional cachectin could be obtained by longer incubation of the cells with LPS, or by restimulation with fresh medium containing LPS. On the contrary, a drop in specific activity was observed when such attempts were made.

No loss in bioactivity was observed over several weeks after storage of the crude preparation at neutral pH at -20°C, or over five days at 4°C. However, bioactivity was noted to be labile at acid pH, with a half-life of 24 hours at 4°C at pH 5.0. Lability at low pH could be diminished by addition of 20% glycerol, For this reason, isoelectric focusing was carried out in glycerol rather than sucrose, and this step was performed as rapidly as possible.

Concentration and Isoelectric Focusing. Bioactivity was associated exclusively with the retentate when pressure dialysis was performed under the conditions described. Recovery after a 50-fold concentration was generally >90%, and was occasionally enhanced as set forth in Table I, below. The data in Table I are from a representative isolation.

TABLE I

| Step | Volume ml | Total Activity U | Total Protein mg | Specific Activity U/mg | Fold purification | Recovery % |
|---|---|---|---|---|---|---|
| Unconcentrated crude | 855 | $2.5 \times 10^7$ | 53.9 | $4.6 \times 10^5$ | --- | 100 |
| Concentrated crude | 54 | $2.7 \times 10^7$ | 38.9 | $6.9 \times 10^5$ | 1.5 | 108 |
| Isoelectric focusing | 94 | $1.5 \times 10^7$ | 3.88 | $3.9 \times 10^6$ | 8.5 | 60 |
| Con A-Sepharose® | 104 | $1.4 \times 10^7$ | 2.78 | $5.0 \times 10^6$ | 11 | 56 |
| Nondenaturing | 8 | $1.1 \times 10^6$ | 0.03 | $1.8 \times 10^7$ | 80 | 2 |

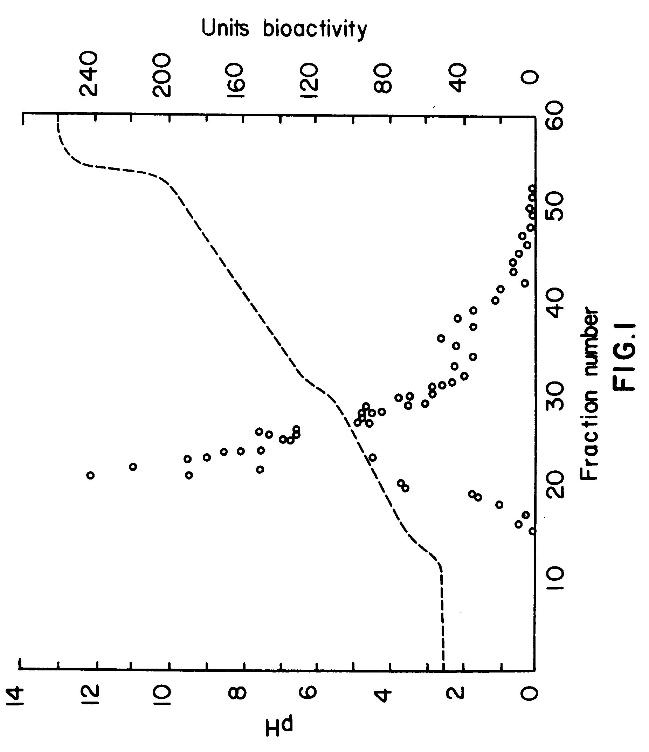

Isoelectric focusing of the concentrated material revealed bioactivity with an isoelectric point of 4.7 (Fig. 1). Ampholytes by themselves do not interfere with the bioassay. Recovery of bioactivity after isoelectric focusing was variable (10-60%) and depended to a large extend upon the total quantity of protein added to the column. The loss of bioactivity during isoelectric focusing appeared to result from coprecipitation of cachectin with other proteins present in the crude concentrate, since poor yields were associated with separations of large quantities of protein and the formation of large amounts of precipitate. Later experiments in which radiolabeled purified cachectin was added to the crude mediator substance as a tracer before isoelectric focusing supported this conclusion. Recovery was maximized by addition of a small sample to the column (50 mg total protein or less), the use of a glycerol gradient, and a short separation time (24 hours or less), with immediate neutralization of samples after fractionation.

Con A-Sepharose® Chromatography. Several protein species were removed by affinity chromatography on Con A-Sepharose® with little or no loss of bioactivity. Use of larger quantities of Con A-Sepharose® resulted in lower recovery of cachectin activity, possibly because of hydrophobic interaction with the resin, since the activity could not be released by addition of 0.1 M $\alpha$-methylmannoside.

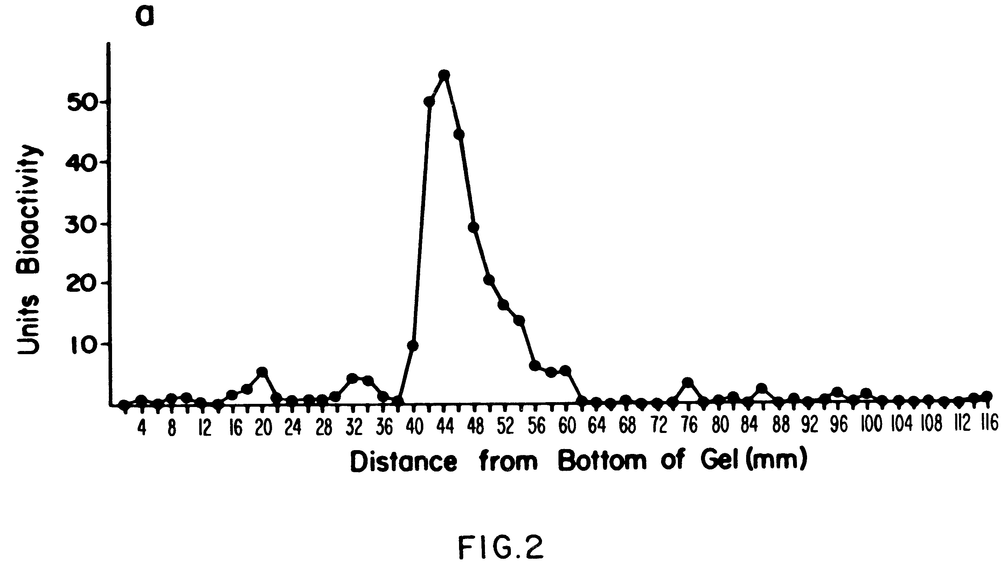

PAGE. After PAGE under nondenaturing conditions, all of the bioactivity was found in one location on the gel, associated with a single protein band (Fig. 2,3). In this step, only 10-20% of the bioactivity could be recovered from the sliced gel, either by electroelution or by allowing diffusion of the protein into dilute ammonium bicarbonate buffer. Longer diffusion times, repeated extraction, and use of larger volumes of buffer had little effect. Recovery was found to be maximized by the application of a highly concentrated sample to the gel, and by the use of purified acrylamide monomer in gel preparation.

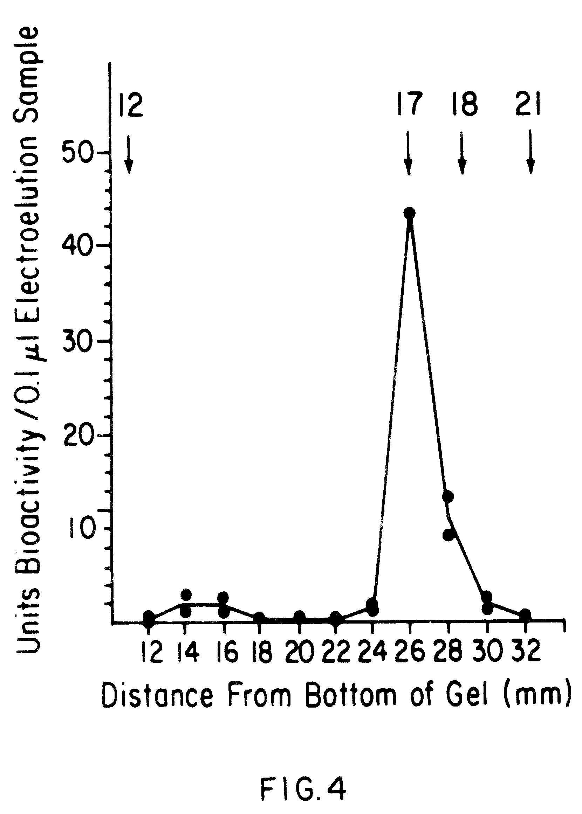

SDS-PAGE Analysis of Electrophoretically Purified Cachectin. The apparent homogeneity of the isolated product was confirmed by electrophoresis in an SDS-PAGE system. The purified protein associated with cachectin activity, applied to SDS gels, yielded a single band of molecular weight 17,000 (Fig. 3). When microgram quantities of purified cachectin were electrophoresed in SDS gels, bioactivity could be recovered from unfixed slices by electroelution. The activity coincided with the presence of the 17,000 molecular weight protein; no bioactivity was obtained from other regions of the gel (Fig. 4).

Molecular Weight of Purified Cachectin. When crude concentrates of cachectin were analyzed by means of Sephadex® G-75 gel filtration, the bioactivity elution profile indicated a molecular weight in >70,000. A molocular weight estimate of 17,000 (dextran scale) was obtained, however, when crude concentrates were separated by gel filtration in the presence of 6 M urea, suggesting the formation of noncovalent multimers or aggregates with other proteins (data not shown).

When purified cachectin was analyzed on SDS gels, both reduced and nonreduced samples exhibited an apparent molecular weight of 17,000. Gel filtration of purified cachectin on Sephadex® G-75 columns provided an estimated molecular weight of 35,000, indicating the presence of noncovalent dimers (data not shown).

Radioiodination and Binding Studies. The purified hormone was radiolabeled with $^{125}$I by the iodogen method. ~70% of the bioactivity was recovered, with an initial specific activity of ~1 x $10^6$ cpm/$\mu$g protein. The labeled material was subjected to SDS-PAGE, and measurement of sliced gels showed most of the radioactivity to be associated with the 17,000 molecular weight protein. Attempts to label the protein using chloramine T resulted in a complete loss of bioactivity.

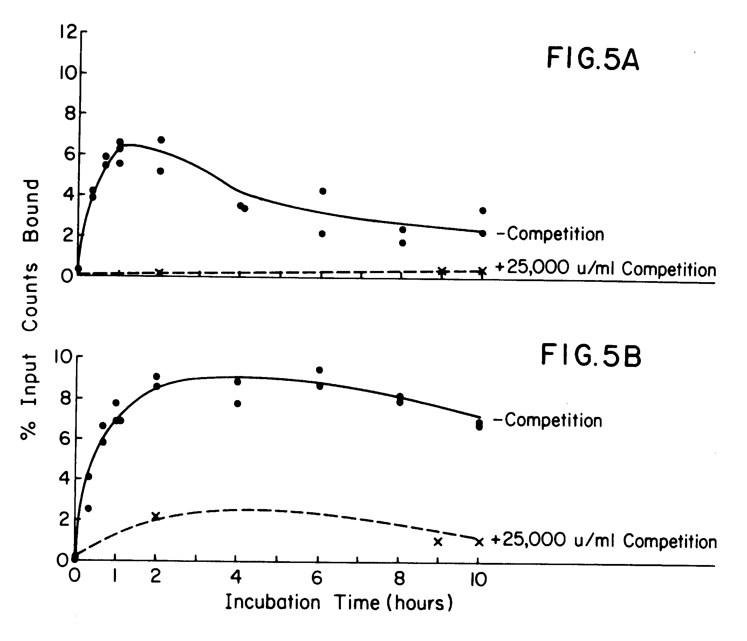

The purified, radioiodinated hormone was rapidly and specifically bound by cultured 3T3-L1 adipocytes at both 37° and 0°C (See Figs. 5A and 5B). Addition of 25,000 U/ml of unlabeled cachectin prevented binding by the labeled protein at all time points examined. Equilibrium was achieved within 2-3 hours under the conditions described. A time-dependent decline in the quantity of label bound was observed at 37°C, possibly reflecting receptor-mediated endocytosis.

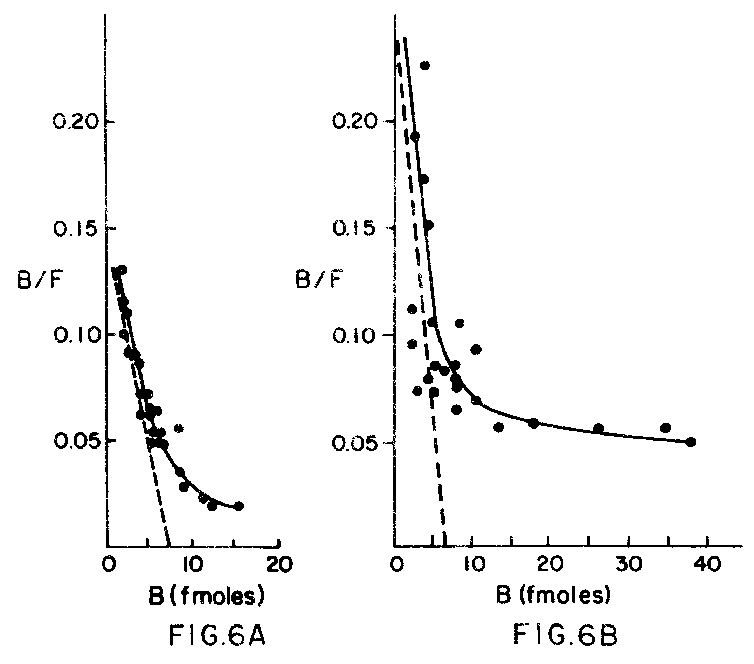

The characteristics of binding were examined by Scatchard analysis (Fig. 6A). The data suggest the existence of ~$10^4$ high-affinity sites per cell, and an association constant (Ka) of 3 x $10^9$. Both differentiated 3T3-L1 cells and undifferentiated "pre-adipocytes" possessed receptors, with similar affinity and density. The muscle cell line C2 also possessed a specific cachectin receptor. Again, ~$10^4$ high-affinity sites per cell and a $K_a$ of 3 x $10^9$ were observed (Fig. 6B). Mouse liver membrane preparations also exhibited specific cachectin binding. On the other hand, erythrocytes and lymphocytes lacked detectable quantities of cachectin receptor (<200 copies per cell).

Assays for IL-1 Activity. In view of its similar size, tissue of origin, and endotoxin inducibility, it appeared possible that the protein isolated was identical to IL-1. The large quantity of cachectin produced by RAW 264.7 cells suggested that this was not the case, particularly since it was found that the cells produced very little IL-1, as measured by leucocyte-activating factor (LAF) activity under the conditions used to elicit cachectin.

Purified cachectin preparations lacked LAF activity when assayed over a wide range of concentrations up to 1 nM, under conditions at which recombinant IL-1 could be detected at $10^{-11}$ M. Moreover, micromolar concentrations of highly purified recombinant IL-1 failed to compete with the radioiodinated cachectin for receptor binding. It therefore appears that cachectin is distinct from IL-1.

DISCUSSION

In the foregoing experiment, the purified protein was isolated from the macrophage-derived mediator and was found to have a monomeric molecular weight of 17,000 on SDS gels, and an isoelectric point of 4.7. Under nondenaturing conditions, the purified protein apparently exists as a dimer.

1 U of bioactivity corresponds to ~2 X $10^{-15}$ mol of isolated monomer. Thus, a 2 pM solution of the modulator material, cachectin is readily detectable by means of the bioassay described. This estimate of specific activity must be considered minimal, since an unknown quantity of the protein may have been rendered biologically inactive during purification.

The isolation of cachectin was facilitated by the use of the murine macrophage cell line RAW 264.7, which allowed large-scale production of the protein for analysis and purification. In addition to being a homogeneous population, the cells could be maintained in serum-free medium during endotoxin stimulation. The RAW 264.7 cell line was generated through transformation of mouse peritoneal cells by Abelson leukemia virus. The cells resemble macrophages morphologically and functionally. They possess receptors for complement and Fc fragments of IgG, and are able to phagocytose neutral red, opsonized RBC, and latex beads. Marked similarity between the cachectin activity produced by RAW 264.7 cells and that produced by thioglycollate-elicited peritoneal macrophages has previously been demonstrated.

SDS-PAGE analysis of culture medium derived from LPS-stimulated and unstimulated RAW 264.7 cells revealed that cachectin is one of the major endotoxin-inducible secretory proteins. It constitutes 1-5% of the total protein secreted by stimulated RAW 264.7 cells, and is clearly visible as a distinct 17,000 molecular weight band on SDS gels. Medium from unstimulated cells completely lacks cachectin bioactivity and the 17,000 molecular weight band. It would appear, based on estimates of the specific activity of purified cachectin, that the 17,000 molecular weight protein accounts for most, if not all, of the bioactivity present in conditioned cell medium. While the possible relationship of the modulator material to other monokines (e.g. interferons, glucocorticoid-antagonizing factor, or other bioactivities produced by macrophages) has not been fully assessed, it is clear from the bioactivity data presented above that the modulator material, cachectin is distinct from IL-1, despite similar molecular weights and isolectric points.

Using labeled cachectin, the existence of specific receptors for cachectin has been demonstrated on differentiated 3T3-L1 cells and preadipocytes, C2 muscle cells, and mouse liver cell membranes. On the other hand, receptors were not detected on mouse-erythrocytes or lymphocytes. The receptor density on 3T3-L1 cells and on C2 cells was ~$10^4$ per cell. The $K_a$, the degree of receptor occupancy, can be determined as a function of cachectin concentration. It would appear that occupancy of as few as 5% of the receptors present on a 3T3-L1 cell (achieved at 2 X $10^{-11}$ M) is sufficient to elicit 70-80% suppression of LPL. This type of relationship has been noted with many other hormone receptor-binding systems.

Although the macrophage is not a characteristic endocrine cell, it can produce the monokine, cachectin, which may be described as a hormone in the classical sense, since it is produced by a specific group of cells, and influences, via a high affinity receptor, the behavior of other cell types. As such, it provides an example of the endocrine capabilities of reticuloendothelial cells. In view of the fact that muscle, liver, and adipose tissues possess receptors for cachectin, a wide range of biological responses is anticipated.

The foregoing results also suggest that cachectin may serve to mobilize the energy reserves of a mammal confronted by acute infection, to meet increased metabolic demands. When chronically evoked, as in parasitic infections, cachectin may contribute to the protein and lipid catabolism that ultimately reduce the host to a state of cachexia.

Further studies conducted after the above experiments sought identify the specific structure of the modulator material cachectin. These studies also reported by Beutler et al. are set forth in Example 2, below.

EXAMPLE 2

When orginally purified from RAW 264.7 (mouse macrophage) cells, cachectin was shown to have a pI of 4.7, a subunit size of 17 kd, and to form non-covalent multimers. A receptor for cachectin was identified on non-tumorigenic cultured cells, and on normal mouse liver membranes.

In the present Example, studies employing a new high-yield purification technique revealed further details of the structure of mouse cachectin. It was observed that a high degree of homology exists between the N-terminal sequence of mouse cachectin and the N-terminal sequence recently determined for human tumor necrosis factor (TNF) and reported in Pennica, D., et al., NATURE, 312:724-729 (1984) and Shirai, T., et al., NATURE, 313:803-806 (1985). Purified cachectin also possesses potent TNF activity in vitro.

The procedures and results of these studies are set forth below.

## MATERIALS AND METHODS

Cachectin bioactivity (LPL suppression) was assayed using 3T3-L1 cells as previously described. TNF activity was measured using the standard cytotoxicity assay with actinomycin D-treated L-929 cells.

A 50-fold concentrate of crude cachectin, derived from lipopolysaccharide-stimulated RAW 264.7 cells, was prepared using a PM-10 membrane in a stirred cell (Amicon Corp.; Danvers, Massachusetts) as previously described in Example 1, above. Before removal of the sample from the ultrafiltration cell, octyl glucoside (Sigma; St. Louis, Missouri) was added to a concentration of 1% (w/v). Addition of a non-ionic detergent was essential in order to achieve high recovery in subsequent steps.

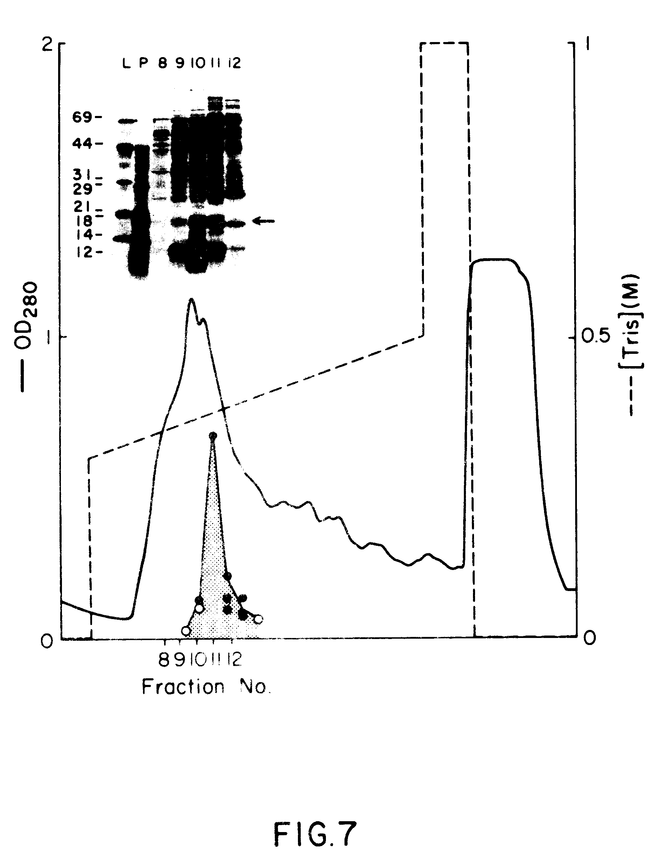

The sample was passed through a 0.22 $\mu$ filter, and applied to a Mono Q (high performance an ion exchange) column (FPLC; Pharmacia; Uppsala, Sweden). Approximately 60% of the total protein passed directly through the column. Cachectin eluted as a sharp peak at a concentration of 0.38 M tris-Cl, pH 7.8 (Fig. 7). Approximately 80% of the hormone was recovered in three fractions.

Mono Q (FPLC Anion Exchange) Chromatography. Crude concentrated RAW 267.4 medium, containing octyl glucoside as a dispersing agent, was applied to the column. Cachectin was eluted using a 0.3 M to 0.5 M tris-Cl (Sigma Chemical Co; St. Louis, Missouri) gradient, run at a flow rate 0.33 ml/min, over 34 minutes. Optical density (280 nm) is indicated by the solid tracing. The dashed line indicates the molarity of tris-Cl buffer used in elution. The crosshatched area indicates the distribution of cachectin bioactivity, expressed in linear units (each point represents the result of a single bioassay). Inset photograph: a silver-stained SDS polyacrylamide gradient gel (10% to 15% acrylamide) showing separation of crude material and successive column fractions 1 $\mu$l per lane). Molecular weight markers are presented in the far left lanes. The arrow indicates the band corresponding to cachectin (MW = 17 kd).

Approximately 60% of the total protein elutes in the non-retained fraction (not shown). The peak of cachectin bioactivity elutes at a concentration of 0.38 M tris-Cl. The three fractions with highest activity were pooled for further purification.

Superose® 12 (FPLC Gel Filtration) Chromatography. Pooled fractions from Mono Q fractionation were lyophilized, redissolved in distilled water, and applied to the column. Chromatography was carried out in 0.1 M ammonium acetate at a flow rate of 0.3 ml/min. A sample volume of 0.2 ml was used in each separation.

The solid tracing represents the OD at 280 nm. The crosshatched bar indicates the presence of cachectin, as assessed by measurement of bioactivity. Arrows indicate the elution position of molecular weight markers applied to the Superose column. Inset photograph: SDS polyacrylamide gradient gel (10% to 15% acrylamide) showing the electrophoretic profile of successive fractions (1 $\mu$l aliquot per lane) derived from Superose® 12 gel filtration. L and P, molecular weight standards; Q pooled Mono Q fractions (starting material for Superose-® 12 separation).

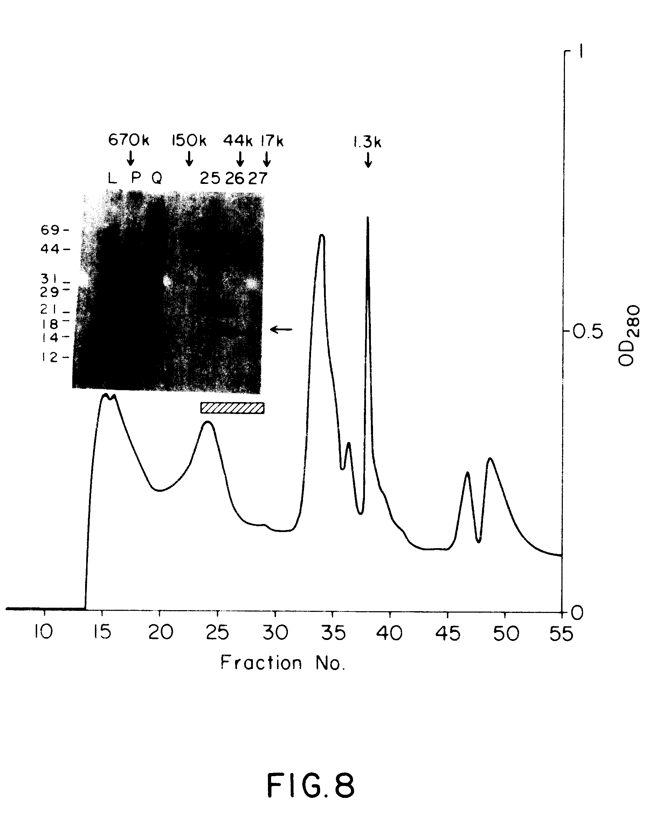

These fractions were pooled, lyophilized, and redissolved in 0.4 ml of distilled water. The sample was then subjected to high performance gel filtration chromatography (Superose® 12; Pharmacia) (Fig. 8).

Cachectin consistently eluted at a position corresponding to a molecular weight of 87 kd, consistent with a pentameric structure. The molecule readily dissociates in the presence of non-ionic detergents. It is unclear whether a pentameric conformation is essential for bioactivity.

The trailing edge of the cachectin peak was estimated to be greater than 95% pure, and contained approximately $10^7$ U cachectin bioactivity/mg protein as did homogeneous solutions produced by means of the older purification method used to prepare the initial mediator substance. TNF activity was assayed in this preparation. Approximately $1.2 \times 10^7$ U/mg protein was measured in purified samples, compared to $3.7 \times 10^4$ U/mg protein in a sample of crude material. The specific activity of purified human TNF has been reported to be $2.9 \times 10^7$ U/mg protein.

The remaining cachectin-rich Superose® 12 fractions, to be used in determinations of the amino acid composition and sequence, were further purified by reverse phase chromatography, using a C8 column (Supelco; Bellafonte, Pennsylvania) as previously described by Pan, Y-C.E., et al., J. CHROMATOG. 297:13-19 (1984). Cachectin eluted at a concentration of 30-40% acetonitrile. 40 pmol of the highly purified protein was hydrolysed with 6N HCl and used for compositional analysis. 200 pmol was used in sequencing. A Waters (Milford, Massachusetts) high performance liquid chromatography (HPLC) system, and an Applied Biosystems (Foster City, California) microsequencing apparatus, model 470 A, were employed in these procedures.

An amino acid composition of mouse cachectin is compared in Table II below, with the composition of human TNF (determined by DNA sequencing), the latter as reported by Pennica, et al. supra. Strong similarities are immediately apparent. Both proteins are, overall, acidic and hydrophobic. Identical numbers of threonine, valine, alanine, histidine, arginine, and cysteine residues are present in each.

TABLE II

| asn/asp | 16 (7/5)* | leu | 17 (18) |
| thr | 6 (6) | tyr | 8 (7) |
| ser | 12 (13) | phe | 6 (4) |
| gln/glu | 20 (10/10) | his | 3 (3) |
| gly | 13 (11) | lys | 10 (6) |
| val | 13 (13) | arg | 5 (5) |
| ala | 13 (13) | pro | ND (10) |
| met | 2 (0) | cys | 2 (2) |
| ile | 5 (8) | tryp | ND (2) |
| ND: not determined | | | |

*Numbers in parentheses refer to the composition established by Pennica, et al., supra. for human TNF.

Likewise, the disposition of the N-terminal amino acid sequence of mouse cachectin (top line) in comparative alignment with the corresponding sequence of human TNF (bottom line), is revealing. Equivocal residues are indicated with a "?". Matching residues are underlined.

Mouse Cachectin

H2N-leu-arg-ser-ser-ser-glu-asn-ser-ser-asp-pro-pro-val-ala

-?-

Human TNF

H2N-val-arg-ser-ser-ser-arg-thr-pro-ser-asp-lys-pro-val-ala

1                                                    10

-his-

Mouse Cachectin   val-val-ala-asn...
Human TNF         val-val-ala-asn...

A review of the above sequence analysis disclosed a striking homology between the N-terminal sequences of mouse cachectin and human TNF. All but five of the first nineteen residues are in agreement. These data, considered together with the cytolytic effect of purified cachectin on L-929 cells, suggested that a single protein species may be responsible for the cachectin and TNF activity of murine macrophage conditioned medium. Furthermore, this protein appears to be highly conserved.

Since the performance of the above investigations and as discussed earlier, the full identity of the modulator has been determined, and a comparison between the fragmentary sequences set forth above and the full sequence shown in FIGURE 23, reveals both similarities and differences. Further analysis of this most recent information is accordingly presently in progress.

It has been previously demonstrated that cachectin is bound by a specific receptor, present in approximately 10,000 copies per cell on cultured 3T3-L1 adipocytes and C2 myotubules. The $K_a$ of the hormone-receptor interaction was determined to be $3 \times 10^9$ $M^{-1}$ by Scatchard analysis. Normal mouse liver membranes also bind cachectin in a competable fashion. A decline in enzymatic activity is observed immediately following addition of the hormone, and apparently results from the specific inhibition of LPL biosynthesis. Net protein synthesis and cell viability remain unaffected.

As discussed earlier, the present modulator material also possesses a number of activities that in combination, form a distinct mosaic of characteristics that define the modulator material. Certain of the activities of the modulator material distinguish it from the known materials.

In particular, the modulator material substantially completely suppresses the anabolic enzyme lipoprotein lipase, and this suppression as demonstrated earlier and herein after is concentration variable. The modulator also prevents the differentiation of fat cells and increases glucose uptake and transport in muscle cells. These latter activities are found in the precursor mediator substance but are absent from Interleukin 1.

By contrast, the modulator material distinguishes its mediator precursor by its lack of ability to inhibit either fatty acid synthetase or acetyl Co enzyme A carboxylase, or to increase the activity of hormone-sensitive lipase. Both the modulator material and its precursor distinguish Interleukin 1 by the lack of leucocyte activator activity and the lack of ability to induce fever in mice or to cause protein degradation.

Apart from the above characteristics, the modulator material also has the ability to cause weight loss in mice and demonstrates the ability to raise antibodies to itself to protect a host against endotoxin-induced shock. All of these activities are illustrated in the examples which follow.

EXAMPLE 3

As demonstrated previously and above, the earlier discovered mediator substance and the present modulator material, cachectin both suppress the activity of the enzyme lipoprotein lipase (LPL). This capability was found in experiments conducted with particular cultured adipocytes (3T3-LI cells), where such inhibition was determined to be virtually complete. In particular, greater than 95% inhibition was reported.

By contrast, the material identified as Interleukin 1 has been noted to exert some inhibition on the activity of lipoprotein lipase, however this activity has been observed to be limited and independent of the concentration of Interleukin 1 administered. Conversely and as noted, in Beutler et al supra., both cachectin and the mediator substance were determined to exhibit the ability to substantially completely inhibit LPL activity. To substantiate these observations, certain experiments were performed with a quantity of a recombinant Interleukin 1 (rIL-1) and with purified cachectin (Beutler et al., supra.) in an effort to confirm this hypothesis. The experimental procedure followed is reproduced below.

MATERIALS AND METHODS

Cell Culture: 3T3-L1 cells were grown to confluence in 24-well Linbro plates and differentiated to form adipocytes as previously described by Mahoney J.R., Jr., et al., J. IMMUNOL. 134:1673 (1985). Briefly, cells were plated at a density of $10^5$/ml in Dulbecco's modified Eagle's medium (DME) containing 10% calf serum. This medium was changed every other day. One week after cultures reached confluence, they were exposed to DME containing 10% fetal bovine serum (FBS), 10 $\mu$g/ml bovine insulin, 0.05 M methylisobutyl-xanthine, and 1.0 $\mu$M dexamethasone for a period of 48 hours. Thereafter, they were maintained in DME containing 10% FBS and 50 ng/ml bovine insulin, changed every other day. DME was purchased from Gibco (Grand Island, New York), and defined calf serum and characterized fetal bovine serum were purchased from Hyclone Laboratories (Logan, Utah). Cells were used for receptor assay four days following differentiation, or for bioassay of LPL suppression one week following differentiation.

Receptor assay: Receptor competition studies were undertaken using the same radiolabelled material previously used in the characterization of the cachectin receptor in Beutler et al., supra. The cachectin receptor assay has been described in detail in Beutler et al. J. EXP. MED., 161:984-995 (1985),and was used here to test the ability of IL-1 to displace radiolabelled cachectin from its specific receptor. In brief, 3T3-L1 cell monolayers, prepared as described above, were overlayed with DME containing 10% FBS and 50 mM HEPES, pH 7.4, and approximately 10-11 $\mu$M radiolabelled cachectin tracer. Unlabelled solutions of IL-1, or cachectin (positive control) were added to proceed unopposed in others. The final volume of each system was 0.2 ml. The reaction was carried out at 0°C for a period of four hours on a rocking shaker. At the end of this incubation, monolayers were washed three times with the same HEPES-buffered medium, solubilized with 1.0 ml of 1 M NaOH, and counted in a Packard gamma counter for a length of time sufficient to yield a standard error of <5%.

Bioassay of LPL suppression by cachectin and IL-1. 3T3-L1 cells, prepared in 24-well Linbro plates as described above, were overlayed with precisely 1.0 ml of DME, supplemented with 10% FBS and 50 ng/ml insulin. Purified preparations of cachectin or recombinant IL-1 were added to each well, and mixed thoroughly. The cells were incubated for a period of 16 hours prior to assay of heparin-releasable LPL activity. Control cell monolayers, which were not exposed to either mediator, were included with all assays.

LPL assay: Medium was aspirated from cell monolayers that had been treated as described above. 0.5 ml of DME containing 10% FBS, 50 ng/ml insulin, and 10 U/ml heparin was then added to each monolayer. After one hour, 75 $\mu$l of medium was assayed for LPL activity according to the method of Nilsson-Ehle and Schotz. Percent LPL suppression (with respect to control monolayers not exposed to either of the mediators) was calculated as previously described in Beutler et al., supra.

Recombinant IL-1: This was the kind gift of Dr. Peter Lomedico (Dept. of Molecular Genetics, Hoffmann-La Roche, Inc.). The monokine (approximately 100 $\mu$M concentration) was provided as a solution in 5 M guanidine HCl. Guanidine HCl, when diluted to submillimolar concentrations, had no influence upon LPL expression in the system described.

Antiserum to IL-1: This antiserum, raised in goat against murine IL-1 was obtained courtesy of Dr. Steven Mizel.

Neutralization of IL-1: Serial two-fold dilutions of antiserum in DME containing 50 mM HEPES, pH 7.4, and 10% FBS, were carried out over the range indicated. Purified recombinant IL-1 was added to a final concentration of 50 pM. After one hour on ice, 15 $\mu$l aliquots of the solution were added to cell monolayers to give a final IL-1 concentration of 0.75 pM. Assays of LPL suppression were performed as usual after a 16 hour incubation.

Purified Cachectin: Preparations of purified hormone were made as described previously in Beutler et al., Supra. Radioiodination was accomplished by means of the iodogen method;an initial specific activity of 1 $\mu$Ci/$\mu$g was achieved, and 70% of the initial bioactivity was recovered.

RESULTS

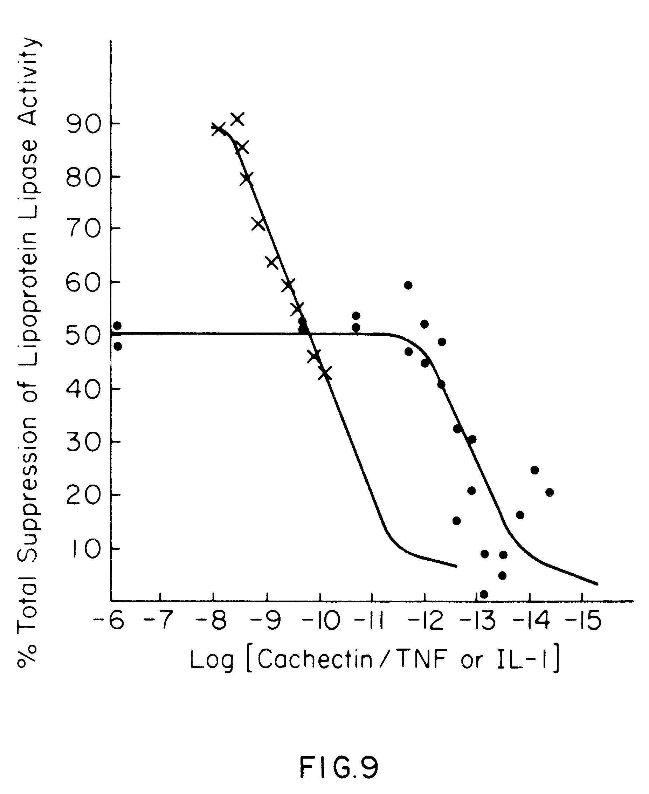

Suppression of LPL activity by purified, recombinant IL-1: The addition of rIL-1 to 3T3-L1 cells partially suppressed LPL activity. As shown in Fig. 9, IL-1 was capable of suppressing LPL activity at concentrations in the femptomolar range. Hence, in the system described, approximately $10^{-18}$ mol of the hormone could be detected. However, even $\mu$M concentrations of the hormone were incapable of reducing LPL by more than 50% under the conditions described, whereas 90 to 95% total suppression could be achieved by nM concentrations of cachectin. Occasional batches of 3T3-L1 cells exhibited greater sensitivity to rIL-1; typically, 50% suppression was the maximum achievable, and in no case did suppression ever exceed 85%.

When cachectin and IL-1 were both added to cultured 3T3-L1 cells, their effects appeared additive and independent. For example, if an amount of IL-1 sufficient to achieve 50% suppression (the maximal response) was added together with an amount of cachectin sufficient to achieve 50% suppression, approximately 75% suppression would be observed (data not shown). A synergistic effect was never observed.

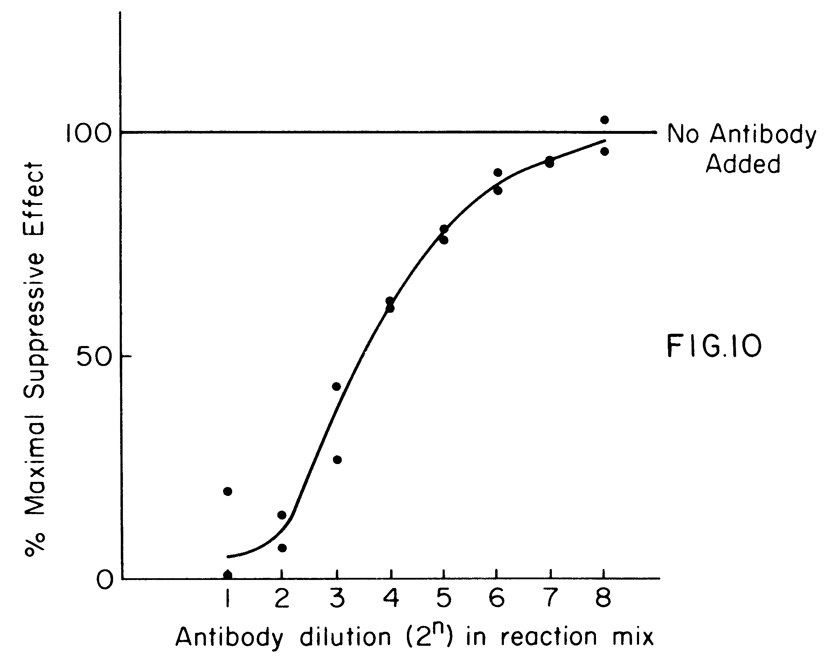

Inhibition of LPL Suppression by Recombinant IL-1 Using Specific Goat $\alpha$IL-1 serum: When 0.75 pM IL-1 was added to cultured 3T3-L1 cells, 80% LPL suppression was achieved (the maximum response in this experiment). Preincubation of the rIL-1 with a specific neutralizing antiserum entirely abrogated this response. The addition of decreasing amounts of the serum resulted in intermediate levels of suppression (Fig. 10). No direct effect of the serum on LPL expression was demonstrable over the range of concentrations used for neutralization (data not shown). It would appear, therefore, that LPL suppression by the rIL-1 preparation used is indeed attributable to the presence of the monokine, and is not due to contaminating bacterial proteins, or to reagents employed in the isolation of IL-1.

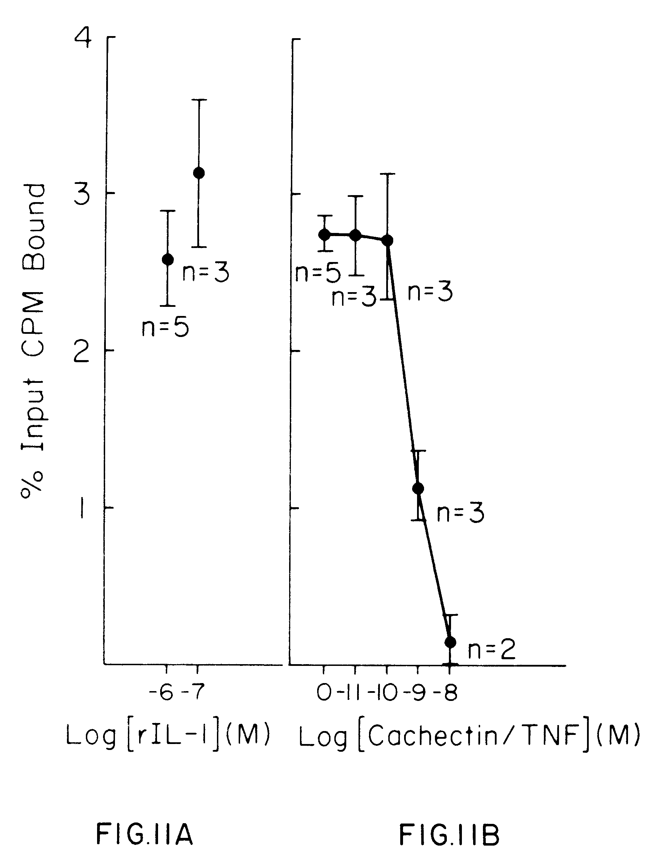

Failure of IL-1 to compete with radiolabelled cachectin for 3T3-L1 receptor sites. As previously described, radiolabelled cachectin is capable of binding to a high-affinity receptor present on 3T3-L1 cells as well as other tissues (Beutler et al., supra.), and is readily displaced by nM concentrations of unlabelled hormone. The addition of 0.1 $\mu$M and 1.0 $\mu$M concentrations of purified recombinant IL-1 caused no significant displacement of the tracer cachectin in this radioreceptor assay (Fig. 11).

Thus it appears that IL-1, while eliciting suppression of LPL in 3T3-L1 cells does so without binding to the cachectin receptor.

It is apparent, however, that the pattern of LPL suppression by Interleukin 1 is distinct from that of either the present mediator or of cachectin, both of which are capable of achieving >95% suppression of LPL in adipocytes. Suppression of LPL by cachectin and by IL-1 is additive and independent; in the presence of a maximally suppressive concentration of IL-1, further suppression can be achieved by addition of cachectin. In addition, as noted above, IL-1 does not appear to share the cachectin receptor.

COMPARISON WITH IL-2. As discussed in Dinarello, supra., the factor identified by the author as Interleukin 1 is distinguished from other factors which are also known to exert some immunoregulatory activities, certain of which have been identified as Interleukin 2. Interleukin 2, formerly known as T cell growth factor, is produced by T cells and has the activity of providing a direct growth signal to lymphocytes. The production of IL-2 is also controlled in some instances in vivo by IL-1. It therefore becomes of interest to determine whether IL-2 exhibits the same activity as IL-1 with respect to LPL suppression.

Accordingly, in Beutler et al., J. EXP. MED., 161:984-995, the authors noted that they had investigated the LPL-suppressing activity of IL-2 and had found that it was non-existent. Apparently, IL-2 does not share this characteristic with IL-1, and is correspondingly likewise distinguishable from both the crude mediator substance and its isolate, the present modulator material, cachectin.

In summary, while the mediator substance, its isolate cachectin and IL-1 all exert a suppressive effect upon LPL, the effect exerted by the mediator substance and cachectin is both structurally and functionally distinct, suggesting that the materials themselves are similarly distinct. Further, all three materials differ from IL-2, so that separate identities must be assumed for each of the materials tested.

## EXAMPLE 4

In Parent Application Publication No. US-A-4603106, the effects of the previously disclosed mediator substance upon the enzymes acetyl CoA carboxylase and fatty acid synthetase were studied, and it was determined that the mediator substance inhibited both enzymes by interfering with their synthesis. In the present experiment, the assay system of Example II of the above application was set up and repeated for each of Interleukin 1 and cachectin. Purified IL-1 and cachectin were prepared as disclosed in Beutler and Cerami, supra. and were used in place of the conditioned medium prepared mouse peritoneal exudate cells that had been cultured in the presence of endotoxin.

Accordingly, 3T3-L1 cells were exposed to each of IL-1 and cachectin, and were incubated for a total of twenty hours, with determinations of acetyl CoA carboxylase and fatty acid synthetase activities made at three, six and twenty hours, respectively. In each instance, the digitonin released cytosolic fractions of the cells incubated with the respective factors displayed no decrease in the activity of either enyzme, and it was therefore determined that neither IL-1 nor the present modulator material, cachectin, had any suppressive effect upon the anabolic enyzmes acetyl CoA carboxylase and/or fatty acid synthetase. In this manner therefore, the present modulator material apparently distinguishes the crude mediator but is similar to Interleukin 1.

## EXAMPLE 5

This example is excerpted from studies conducted by one of the present Applicants and co-workers which have been prepared in unpublished findings in an article entitled "A Murine Model for Cachexia in Chronic Disease: Endotoxin Induced Macrophage Secretory Protein Activates Catabolism in 3T3-L1 Fatty Fibroblasts". In this study, the role of the crude mediator substance in the inhibition of adipose cell metabolism was further investigated, by focusing on the effect that the mediator might have upon the catabolic activity of the mobilization and release by the cells of triacyl glycerol. This lipolytic activity was measured directly and by reference to the activity of both hormone sensitive lipase and cAMP and it was found that the mediator had a significant effect on the rate and extent of lipolysis and the activity of hormone sensitive lipase, but that cAMP levels and activity were virtually unaffected. The procedures followed in this investigation are reproduced below.

## MATERIALS AND MISCELLANEOUS METHODS

The various reagents, materials and procedures were utilized in accordance with earlier reported work in this area. Thus, isoproterenol, ACTH, [$^{32}$P] Pi, LPL and mouse peritoneal macrophages were all either obtained or prepared from conventional sources. Radioimmunoassays for cAMP were performed as reported by Watkins et al., J. BIOL. CHEM., 257:14719 (1982).

3T3-L1 Cell Culture. 3T3-L1 preadipocytes were cultured as previously described by Pekala et al., PRO. NATL. ACAD. SCI. USA, 80: 2743 (1983), in Dulbecco's modified Eagle medium containing 10% fetal bovine serum. Differentiation was induced two days post confluence by supplementing the medium with 0.5 mM isobutyl-methylxanthine, 1 $\mu$M dexamethasone, and 10 $\mu$g of insulin per ml. Forty-eight hours later this medium was withdrawn and replaced with medium supplemented only with 10% fetal bovine serum and insulin. After four days when greater than 90% of the cells were expressing the adipocyte phenotype, the medium was changed and cells were maintained in the absence of insulin for fourteen days.

Lipolysis. Lipolysis was assessed as glycerol release as per Pinter et al., ARCH. BIOCHEM. BIOPHYS. 121:404 (1967). Cells were washed with Krebs Ringer phosphate buffer with 2% bovine serum albumin (Fraction V), pH 7.4, then incubated in 2.0 ml of the same medium for one to two hours at 37°C. Incubation was terminated by withdrawal of the medium and adjusting it to 10% trichloroacetic acid. The mixture was centrifuged at 2,500 rpm for ten minutes and glycerol content of the supernatant determined using a coupled enzyme system after extraction of the trichloroacetic acid with ether: A sample (0.167 ml) was added to a cuvette containing 0.14 mM ATP, 1.2 mM phosphoenol pyruvate, 0.20 mM NADH, 1.5 units glycerokinase (Candida mycoderma), 1.35 units pyruvate kinase (rabbit muscle), and 2.8 units lactate dehydrogenase (beef heart). The amount of glycerol in the sample is proportional to the NADH oxidized. Glycerol release is reported as total nmol released per unit time either per $10^6$ cells or per mg protein. Under the above conditions glycerol release was constant for at least three hours.

Effect of the Mediator or Isoproterenol on Glycerol Release. Conditioned medium (400 $\mu$l) from endotoxin-treated mouse peritoneal macrophages was added for various periods of time to the culture medium (3.5 ml) of differentiated 3T3-L1 cells (4.2 x $10^6$ cells/dish). This quantity is sufficient to suppress the activity of lipoprotein lipase by 90% after fifteen hours. At the end of the incubation, the medium was removed, the monolayers washed twice with Krebs-Ringer phosphate buffer containing 2% BSA and glycerol release monitored for one to two hours.

In studies performed with isoproterenol, monolayers were washed twice with Krebs-Ringer phosphate buffer containing 2% BSA and incubated in the same buffer. Saturating levels of isoproterenol (3 $\mu$M) were added and glycerol release into the incubation mixture was monitored for one to two hours.

Preparation of Cell Extracts and Assay of the hormone Sensitive Lipase. A soluble cellular extract containing the hormone sensitive lipase was prepared as described by Kawamura et al., PROC. NATL. ACAO. SCI. USA, 78:732 (1981). First, the medium was changed and the monolayers were incubated with medium containing heparin (10 units/ml) for sixty minutes. The medium was removed and the monolayers were washed twice with Dulbecco's phosphate-buffered saline, pH 7.4, and once with 10 mM Tris-HCl/ 1 mM EDTA/0.25 M sucrose, pH 7.4. Cells were scraped and centrifuged at 1000 x g for one minute. The packed cells were homogenized in 2 volumes of 10 mM Tris-HCl pH 7.4, 1 mM EDTA, 0.25 M sucrose and centrifuged at 40,000 x g for one hour. The supernatant was applied to a heparin-Sepharose® affinity column to adsorb lipoprotein lipase. The effluent contained lipolytic activity that was not activated by the addition of serum, indicating the absence of lipoprotein lipase.

Samples of the extract (0.07 ml) were incubated in 5 mM magnesium acetate/0.5 mM ATP/ 0.01 mM cAMP/ 1 mM EDTA/ 25 mM Tris HCl, pH 8 in a total volume of 0.1 ml for five minutes at 30°C to activate the lipase. Assay was initiated by addition of substrate mixture (0.7 ml) containing 0.3 mM [$^3$H]-triolein (7.5 x $10^5$ cpm/$\mu$mol), 50 mM sodium phosphate, bovine serum albumin 5 mg/ml, and 2 mM EDTA, pH 7.0. After incubation for thirty minutes, 3 ml of extraction mixture composed of methanol, chloroform, and heptane (1.41:1.25:1.00) were added. Tritiated oleic acid was separated from esterified oleate by liquid/liquid partition and radioactivity was determined as described by Kawamura et al., supra.

RESULTS

Effect of the Mediator Protein on Cellular Lipolysis.

Previous work had demonstrated a marked decrease in the weight of the epididymal fat pads of mice that had been exposed to either endotoxin or conditioned medium from endotoxin treated macrophages (Kawakami, M., Pekala, P.H., and Cerami, A., unpublished data). This observation was consistent with an activation of lipolysis, leading to a depletion of stored triacylglycerol occurring coincident with the suppression of lipogenesis that was originally disclosed withe respect to crude mediator substance. During a fifteen hour exposure to the mediator, lipolysis measured by glycerol release, increased gradually to approximately 150% over basal. The time frame required for maximum activation of glycerol release is similar to that observed for maximal suppression of the membrane associated lipoprotein lipase. After seventeen hours of exposure to the mediator, hormone-sensitive lipase activity, assayed in soluble cell extracts, was increased 60-70% as shown in Table III, below.

## TABLE III

### Effective of a Macrophage Mediator on the Hormone-Sensitive Lipase of 3T3-L1 Fatty Fibroblasts

Cells were induced to differentiate and maintained as described in Experimental Procedures. A soluble fraction containing the hormone-sensitive lipase was prepared from control cells and cells that had been exposed for seventeen hours to conditioned medium from endotoxin-treated mouse peritoneal macrophages. The extract was either assayed directly or incubated for five

minutes in the presence of cAMP and ATP prior to assay as described in Experimental Procedures. Values are the mean $\pm$ standard deviation of four experiments.

Hormone-Sensitive Lipase Activity
nmol/hr/mg protein

| | Control Cells | Mediator-Treated Cells |
|---|---|---|
| None | 146 $\pm$ 18 | 240 $\pm$ 42 |
| cAMP | 304 $\pm$ 54 | 263 $\pm$ 37 |

Incubation of control extracts in the presence of cAMP and ATP resulted in a doubling of lipase activity. No such stimulation was observed, however, when extracts prepared from mediator treated cells were incubated in the above manner. In the same experiment, cells exposed to saturating levels of the $\beta$-adrenergic antagonist isoproterenol (3 $\mu$M) [Watkins et al., J. BIOL. CHEM., 257:14719 (1982)], exhibited a maximum in glycerol release of 310 nmol/hr/mg protein. This compares to a maximum in mediator treated cells of 315 nmol/hr/mg protein, as set forth in Table IV, below.

Table IV
Maximum Rate of Glycerol Release From
3T3-L1 Fatty Fibroblasts

3T3-L1 cells were exposed to the mediator for seventeen hours or isoproterenol for one hour and glycerol release determined as described in Experimental Procedures. Values are the mean $\pm$ S.D. of four experiments.

| Treatment | nmol/h/mg protein | nmol/h/10$^6$ cells |
|---|---|---|
| Control | 118 $\pm$ 15 | 61 $\pm$ 10 |
| Mediator | 315 $\pm$ 18 | 164 $\pm$ 10 |
| 3 M Isoproterenol | 310 $\pm$ 25 | 162 $\pm$ 12 |

As a comparison, purified cachectin prepared in accordance with the present disclosure, and IL-1 were each incubated with 3T3-L1 cells that had been induced to differentiate, under the same conditions described with reference to the medium derived from endotoxin-treated mouse peritoneal macrophages. Thereafter soluble fractions containing the hormone sensitive lipase were prepared in identical manner and were assayed directly whereupon it was found that hormone sensitive lipase activity as expressed in nmol/hr/mg protein was the same as that of the control cells set forth in Table IV, above.

From the above data, it can be concluded that, while the crude mediator substance appears to stimulate lipolysis and the activity of hormone-sensitive lipase, this activity is not demonstrated by either the present modulator material or the known factors identified and represented by IL-1.

EXAMPLE 6

This example derives from an investigation by Torti et al., supra. that was conducted in order to elucidate the mechanisms by which the previously discovered mediator substance might function to inhibit lipogenic enzyme activity in adipocytes. The effects of conditioned media of endotoxin stimulated macrophage cultures on a stable adipogenic cell line (TA1) were examined using cDNA clones of genes whose expression is increasing during adipocyte differentiation as disclosed in Chapman et al., J. BIOL. CHEM., 259:15548-15555 (1984). It has been demonstrated that the mediator reversibly and specifically inhibits the developmental expression of adipose inducible genes and that this occurs by inhibition of transcriptional activation of these genes. Further, when mature adipocytes are exposed to the mediator, the mRNAs of these genes induced during adipogenesis diminish and rapidly approach predifferentiated levels.

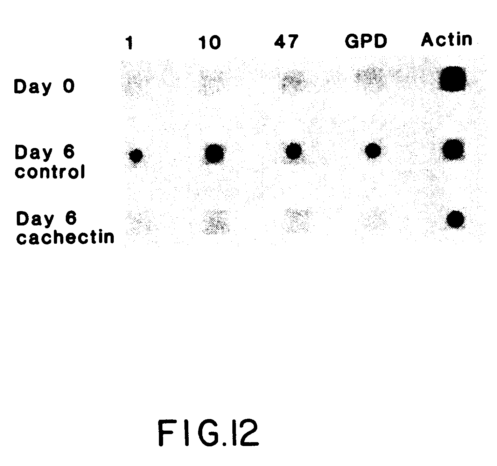

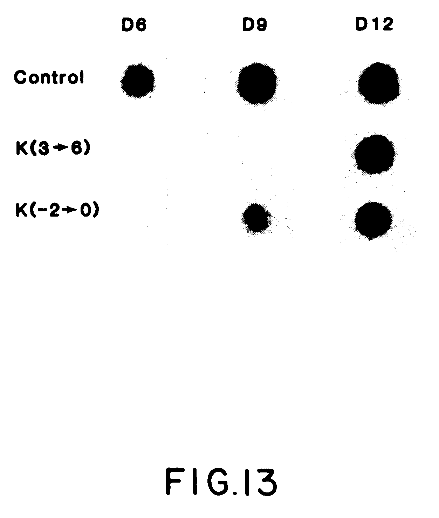

TA1 cells develop a typical adipocyte morphology approximately three days after confluence in tissue culture monolayers as described in Chapman et al., supra. In parallel with this morphological evidence of differentiation, several genes whose expression is first evident after differentiation have been identified (clones 1, 10, 28, 47). These adipose inducible mRNAs are expressed largely or completely due to transcriptional activation. To assess what influence the present mediator might have on the coordinate induction of these adipose genes, the mediator was added to preconfluent TA1 cells and to cells the day they reached confluence. Total RNA isolated from these and control cells six days after confluence was probed with radiolabeled cDNAs of genes whose expression was observed in adipocytes, but not in preadipocytes. As seen in Figure 12, mediator treatment prevents accumulation of adipose-inducible mRNAs. Lipid accumulation is also completely inhibited by mediator treatment. TA1 adipocyte cell cultures treated with the mediator have been maintained for as long as twenty-three days without the appearance of neutral lipid evident by sudan III staining (data not shown). However, on removal of the mediator from the media, adipocyte morphology returns as does the expression of adipose inducible genes e.g., clone 1 (Figure 13).

This inhibition of gene expression is not a general effect of the mediator; for example, as evident from Figure 12, the level of $\beta$-actin mRNA is unaffected. In addition, treatment of preadipocyte cultures with the mediator does not affect cell growth or viability. In a series of experiments similar in design to those in Figure 12, cellular proliferation was determined by cell counting and by $^3$H-thymidine incorporation. Control and mediator treated cultures were indistinguishable in cell number increase and $^3$H-thymidine incorporation decrease at confluence. Cell viability, as determined by trypan blue exclusion and a clonal growth assay, was also equivalent in control and mediator treated cells (data not shown).

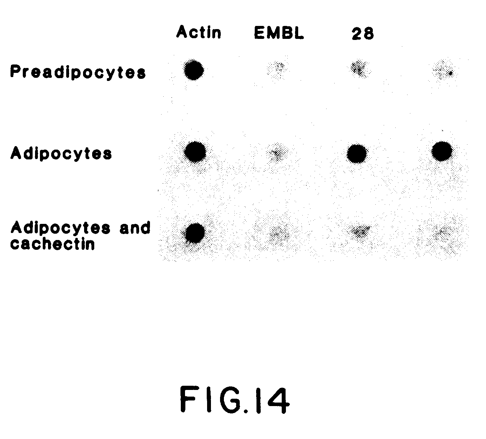

To evaluate whether the mediated inhibition of adipose-specific mRNA accumulation is transcriptionally regulated, nuclear transcription assays were performed. Figure 14 shows that the normal developmental increase in gene transcription for clones 1 and 28 is inhibited by the mediator. Subsequently, similar results have been obtained for cDNAs from glycerophosphate dehydrogenase (gift of B. Spiegleman) and clone 47 (data not shown). In additional experiments, the decrease in GPD mRNA level after mediator addition parallels decreases in GPD enzyme activity (data not shown). Mediator treatment, however, does not cause a nonspecific reduction of transcriptional activity: the transcription rates of $\beta$-actin in preconfluent cells, differentiated adipocytes, and cells treated with the mediator during differentiation are equivalent (Figure 14).

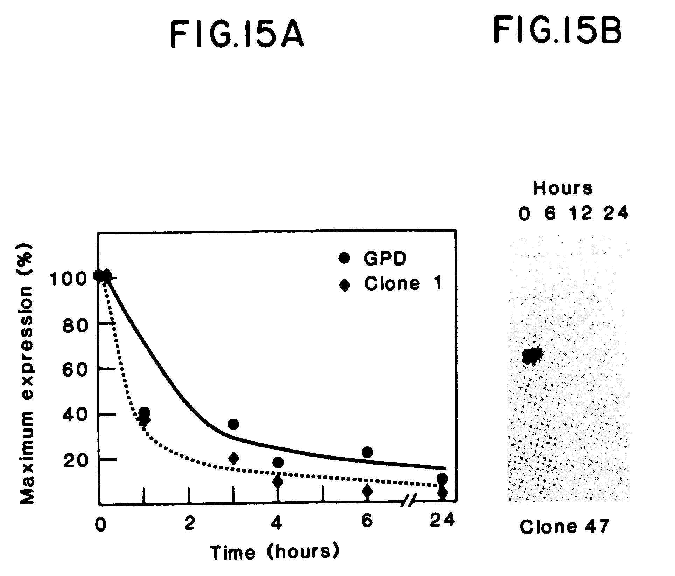

In adult mammals, adipocytes undergo little or no proliferation. Thus the inhibition of adipose gene expression during differentiation when the mediator is added to preconfluent TA1 cells, although useful in understanding how these adipose genes are coordinately regulated in development, probably does not represent a realistic model of mammalian cachexia. To more faithfully utilize these cells to model the in vivo situation, mature adipocyte cultures were established and the mediator was added to these cells. After four to six days of exposure to the mediator, most cells lose their neutral lipid. In typical experiments, 70-80% of cells would be laden with large lipid droplets when the mediator was first added to these cultures; six days later, approximately 10% of cells would have identifiable triglycerides on sudan III stains. As seen in Figure 15, alterations in adipose-specific RNAs occur more rapidly than lipid mobilization. By twelve to twenty-four hours after addition of mediator to mature TA1 adipocytes, a greater than 90% decrease in the levels of such RNAs is observed. Thus a dramatic and coordinate alteration in gene expression appears to be of primary importance in the mediator-induced mobilization of lipid.

The Figure legends describing the specific procedures from which the data was gathered, are set forth below.

Figure 12. TA1 cells, a stable adipogenic cell line derived from 5-azacytidine treatment of 10T/2C18 cells were grown in Eagles Basal Medium supplemented with 10% heat inactivated fetal calf serum. $10^{-6}$ M dexamethasone was present in media for the first three days after confluence, and 5 $\mu$g/ml bovine insulin for the first six days after confluence. Conditioned media from macrophage cell line RAW 264 was first added to preadipocyte cultures two days prior to confluence at a concentration of 10 $\mu$ l/ml, which inhibits 90% of lipoprotein lipase activity in cultured adipocytes. Cells were fed with resupplementation of hormones at Day 0 (confluence) and Day 3. Cells were harvested at Day 6. Total RNA was isolated by the method of Chirgwin et al., BIOCHEMISTRY, 18:5294-5299 (1979), and applied to nitrocellulose in a dot blot apparatus (BRL). Nick translated cDNA clones of genes whose expression is seen only in differentiated TA1 adipocytes (clones 1, 10, 47, and glycerophosphate dehydrogenase), as well as a $\beta$-actin cDNA clone (gift of P. Gunning and L. Kedes) were used to probe these filters under hybridization conditions previously described in Chapman et al., supra. Filters were washed, then exposed to XAR5 film at -70°C with an intensifying screen.

Figure 13. Dot blots were performed as described in Figure 12. Mediator (K) was present in the media at the times indicated. RNAs were analyzed with clone 1 cDNA at six, nine and twelve days after confluence.

Figure 14. Transcription assays were performed using the method described by Vannice et al., PROC. NATL. ACAD. SCI., USA, 81:4241-4245 (1984), and Israel and Whitlock, J. BIOL. CHEM.,259:5400-5402 (1984), as modified by Knight et al. for adipose cells. Cultured cells were chilled to 4°C, media aspirated and washed with phosphate-buffered saline. One ml of hypotonic buffer (20 mM Tris/HCl, pH 8.0, 4 mM MgCl$_2$, 6 mM CaCl$_2$, 0.5 mM dithiothreitol) was added to plates. After five minutes, 1 ml of lysis buffer (0.6M sucrose, 0.2% NP-40, and 0.5 mM dithiothreitol) was added, and cells were scraped from the tissue cultures dishes. After Dounce homogenization, nuclei were pelleted at 500 g, washed once in resuspension buffer (0.25 M sucrose, 10 mM Tris/HCl, pH 8.0, 10 mM MgCl, 1 mM dithiothreitol) repelleted, then resuspended in 50 mM HEPES pH 8.0, 90 mM NH$_4$Cl, 5 mM MgCl, 0.5 mM MnCl$_2$, 2mM dithiothreitol, 0.1mM EDTA, 0.4 mM each of ATP, CTP, GTP, 10% glycerol and 10 $\mu$g/ml bovine serum albumin. Nuclei were incubated with $^{32}$P-UTP (600Ci/mmole, ICN) at a concentration of 2 mCi/ml for forty minutes at 25°C with gentle shaking).

RNA was harvested from nuclei as described by Smith et al. CELL, 15:615-626 (1978) and modified by Knight et al, and hybridized to linearized cDNAs which had been applied to nitrocellulose filters and baked for two hours at 80°C in a vacuum oven. Filter prehybridization and hybridization conditions were those of Friedman et al., CELL, 38:745-755 (1984). Hybridizations were performed for three days at 42°C with approximately 15 x $10^6$ cpm per reaction mixture applied in 150 $\mu$l volume to dots of adipose cDMAs for clones 1 and 28, as well as $\beta$-actin and pEMBL plasmid controls.

Figure 15A. Ten $\mu$l/ml of the mediator substance was added to Day 6 adipocyte cultures differentiated as described in Figure 12. Total RNA was isolated from cells at the indicated times after exposure to the mediator, and applied to nitrocellulose with a dot-blot apparatus (Schleicher and Schuell). Filters were probed with the indicated cDNAs, washed, autoradiographed and scanned using a Hoeffer GS300 densitometer attached to a reporting integrator (Hewlett-Packard). Points shown were normalized for differences in amount of applied RNA using a cDNA probe made to total cellular RNA.

Figure 15B. For these Northern analyses, 12 $\mu$g of total RNA was brought to a final concentration of 2.2M formaldehyde, 30% formamide, 10 mM NaH$_2$PO$_4$, pH 7.0, and heated for fifteen minutes at 56°C. Samples were electrophoresed in a 1.0% agarose formaldehyde gel with the final concentration of 2.2M formaldehyde, 20 mM MOPS, pH 7.0, 5.0 mM sodium acetate and 1 mM EDTA as decribed in Maniatis et al., "Molecular Cloning: A Laboratory Manual"; (Cold Spring Harbor, New York: Cold Spring Harbor Laboratory), pp. 202-203 (1982). Gels were washed in distilled water for 3 minutes, followed by two 30 minute washes in 10 mM NaPO$_4$ (pH 7.4) and 1 mM EDTA, then transferred to nitrocellulose. The insert shows a typical Northern analysis probed with clone 47 DNA.

The experiments described above were carried out with both crude conditioned media of endotoxin stimulated macrophages corresponding to the earlier discovered mediator substance and with the protein cachectin recently purified to homogeneity by Beutler et al., supra. Comparative tests were performed with Interleukin 1, (mouse recombinant IL-1, gift of P. Lomedico, Hoffman-LaRoche) and it was found that IL-1 does not inhibit adipocyte differentiation. Thus both the mediator substance and the present modulator material cachectin exhibit a suppressive effect upon adipocyte differentiation.

EXAMPLE 7

In this experiment, the activity of the uptake and metabolism of glucose by muscle cells was studied. It was theorized that the same factor that would promote the catabolic activities attendant to shock that occur responsive to invasive stimuli, might also contribute to or have some causal effect upon the consumption of glucose that leads to the hypoglycemia also present during shock.

In support of this hypothesis, it was observed that a crude preparation of the mediator substance injected intraperitoneally caused hypoglycemia in endotoxin resistant C3H/HeJ mice, and that small amounts of both the crude mediator and the present modulator cachectin, increased 2-deoxyglucose transport. It was therefore suspected that the crude mediator and the present modulator might play a role in the regulation of metabolism responsive to infection, and a comparative study was conducted including the crude mediator substance, the present modulator, cachectin, and including Interleukin 1, to measure the uptake and transport of glucose in L6 muscle cells. The procedures and results follow below.

MATERIALS

Endotoxin (lipopolysaccharide) from E. Coli 0127:B8 isolated by the method of Westphal was purchased from Difco Labs (Detroit, Michigan). Cell culture medium and calf serum were obtained from GIBCO Labs (Grand Island, New York) and fetal calf serum from Sterile Systems, Inc., (Logan, Utah). 3-isobutyl methylxanthine was purchased from Aldrich Chemical (Milwaukee, Wisconsin); Dexamethasone and the CPK assay kit was from Sigma Chemical Company (St. Louis, Missouri), insulin was from Eli Lilly (Indianapolis, Indiana); glycerol tri [9,10(n)-$^3$H] oleate, U-$^{14}$C-glucose and 2-deoxy-D[1-$^3$H] glucose was from Amersham Corp. (Arlington Heights, Illinois), 2 methyl(3H)isobutyric acid was from New England Nuclear (Boston, Massachusetts); triolein was from Nu Check Prep, Inc. (Elysian, Minnesota); acetyl pyridine adenine dinucleotide and lactate dehydrogenase from Boehringer-Mannheim (Indianapolis, Indiana).

METHODS

3T3-L1 Cell Culture

3T3-L1 preadipocytes were cultured as previously described in Dulbecco's modified Eagle's medium (DME medium) containing 10% fetal calf serum. Differentiation leading to the adipocyte phenotype was induced. Two days after confluence, the medium was supplemented with 0.5 mM isobutyl-methylxanthine, 1 $\mu$M dexamethasone and 10 $\mu$g of insulin per ml. Forty-eight hours later, the medium containing isobutyl-methylxanthine, dexamethasone and insulin was withdrawn and replaced with medium containing insulin at a reduced concentration of 50 ng per ml.

L6 Cell Culture

The myoblast cell line, L6, was kindly provided by Dr. David Schubert, Salk Institute, San Diego, California. The cells were maintained in 10% fetal bovine serum-DME at 37°C in 5% CO$_2$, trypsinized by standard procedures and plated in densities of 5 x 10$^3$ cells/ml. Confluence was reached after one week. Differentiation to myotubes was facilitated by treatment with insulin (10 $\mu$g/ml) for twenty-four hours after cells had reached confluence.

Preparation of peritoneal exudative cells and mediator substance

Peritoneal exudate cells were obtained by peritoneal lavage from NCS/SPF mice (25-33g; Rockefeller University Breeding Colony) which had been injected intraperitoneally with sterile Brewer's thioglycollate medium (Difco Labs, Detroit, Michigan; 3 ml per mouse) five days prior to harvest. The exudate cells obtained using this procedure are primarily macrophages with some contaminating lymphocytes.

The cells (4 x 10$^5$ cells per cm$^2$) were incubated in serum-free RPMI-1640 medium for three hours after which nonadherent cells were removed by washing three times with normal saline. Cells adhering to the dish were primarily macrophages. These cells were further incubated in serum-free RPMI-1640 medium in the presence or absence of 5 $\mu$g per ml of endotoxin for in vivo use or 0.1 $\mu$g/ml for in vitro use for twenty hours. The culture medium was removed after incubation and centrifuged at 1000 x g for five minutes at 4°C. The supernatant of conditioned medium obtained from cells exposed to endotoxin contained the mediator substance. No difference in activity was noted after storage of the conditioned medium for one

25

month at -80°C.

## 2-Deoxyglucose transport

The accumulation of 2-deoxyglucose in L6 cells was measured by a modification of the procedure for 3T3-L1 cells. L6 cells were grown to confluence in 6 cm tissue culture dishes and used at confluence for assays in myoblasts or differentiated with insulin for assays in myotubes. Macrophage mediator, partially purified mediator or lipopolysaccharide were added approximately twenty-four hours or as designated prior to washing the plate three times with 2 ml of Krebs-Ringer-HEPES (128 mM, NaCl, 5.2 mM KCl, 1.4 mM $CaCl_2$, 1mM $MgSO_4$, 10 mM HEPES, pH 7.4) (KRH). Insulin and/or cytochalasin was added as indicated and the plates incubated thirty minutes prior to the addition of 2-deoxy-$^3$H-glucose (2 $\mu Ci/\mu M$, 02 mM). After ten minutes the plates were washed three times with cold (4°C) 25 mM glucose KRH. The buffer was aspirated and 1.5 ml 10% sodium dodecyl sulfate was added to the plate and allowed to stand at room temperature for thirty minutes to solubilize the cells. The cells were removed from the plate by scraping and suspended with a pipette. The resulting mixture was sonicated at an intermediate power setting on a Branson Sonicater twice for ten seconds and analysed for radioactivity by liquid scintillation counting.

Purified IL-1 was obtained as in the previous experiments and was incubated with L6 cells in the same manner described above with reference to the mediator and its isolate.

## RESULTS

In general, both the mediator substance and its isolate increased 2-deoxyglucose transport by L6 myoblasts and tubules, as much as seven-fold in the instance where high doses of the mediator were used, also establishing that this effect is dose dependent. By comparison, the medium wherein IL-1 was present had no effect on transport.

For example, the measurement of 2-deoxyglucose uptake per ten minutes of incubation taken at the twenty-four hour interval showed uptake by control cells to be 12.5 ± 3.8, with purified cachectin showing 30.3 ± 0.01, while the cells in contact with the IL-1 medium indicated -3.4 ± 0.26. For further reference, a sample with the known glucose transport protagonist, insulin, was incubated with the cells for twenty-four hours and stimulated 2-deoxyglucose uptake to 53.9 ± 3.4. Thus, while the crude mediator substance and the present isolate may not affect glucose uptake to the same extent as insulin, at least in this study, it is clear that both materials are significant stimulators of glucose transport and can be clearly contrasted with IL-1 which lacks this ability.

To conclude, the above experiment indicates that both the present modulator cachectin, and its precursor mediator, possess the activity of stimulating glucose transport in muscle cells, while Interleukin 1 does not.

## EXAMPLE 8

In the continuing effort to identify the activities and properties of both the crude mediator substance and its isolate, cachectin, an investigation was made of the weight loss associated with the administration of the mediator and its isolate in vivo, by observing the effects on loss of body mass and food intake in the C3H/HeJ (endotoxin resistant) mouse.

A comparison was also made herein as to the effects of the respective factors on fever production. From a review of the literature outstanding with respect to Interleukin 1, and in particular the article by Dinarello discussed herein supra., IL-1 was found to cause fever in mice. See Dinarello supra., pages 65-66 and earlier authorities cited therein. The data and results both as to weight loss and as to fever induction are set forth below.

## MATERIALS AND METHODS

### Preparation of Macrophage Mediator

Peritoneal exudate cells were obtained by peritoneal lavage of NCS or C3H/HeN mice (male, 20-25 g) injected six days previously with 3 ml of sterile Brewer's thioglycollate medium and washed with saline solution as previously described by Kawakami et al., J. EXP. MED. 154:631 (1981). The cells (2 x 10⁶ cells/$\mu$l) were resuspended in RPMI 1640 (Gibco) and placed in 10 cm petri dishes for three hours. Non-adherent cells were removed by washing three times with the same medium. The remaining, adherent macrophages were incubated for twenty to twenty-four hours in serum free RPMI 1640 medium containing 5 $\mu$ g/ml of endotoxin and 50 $\mu$g/ml of gentamicin sulfate. The macrophage culture medium was collected, centrifuged, and the solution stored at -78°C. Protein content of the preparation was 15-18 $\mu$g/ml with a specific activity of about 300 U/mg. The unit was determined as the quantity of macrophage factor capable of 50% suppression of lipoprotein lipase activity on differentiated 3T3-L1 cells in the defined condition. Macrophages not activated by endotoxin did not show lipoprotein lipase suppression activity.

The solution of macrophage secretion product was also concentrated with an Amicon Concentrator using a PM 10 membrane. Three concentrated solutions were prepared (two, five, and ten times more concentrated than the original solution for injection in animals).

Mediator Injections

C3H/HeJ mice (male, 18-25 g) received two intraperitoneal injections daily of the solutions described above. Food intake was measured by using metabolic cages. Body mass was measured by weighing the animals daily. During this time, the temperature of the animals was also monitored daily.

FIGURE LEGENDS

The following figure legends describe the data on weight loss and the manner in which it was generated and gathered.

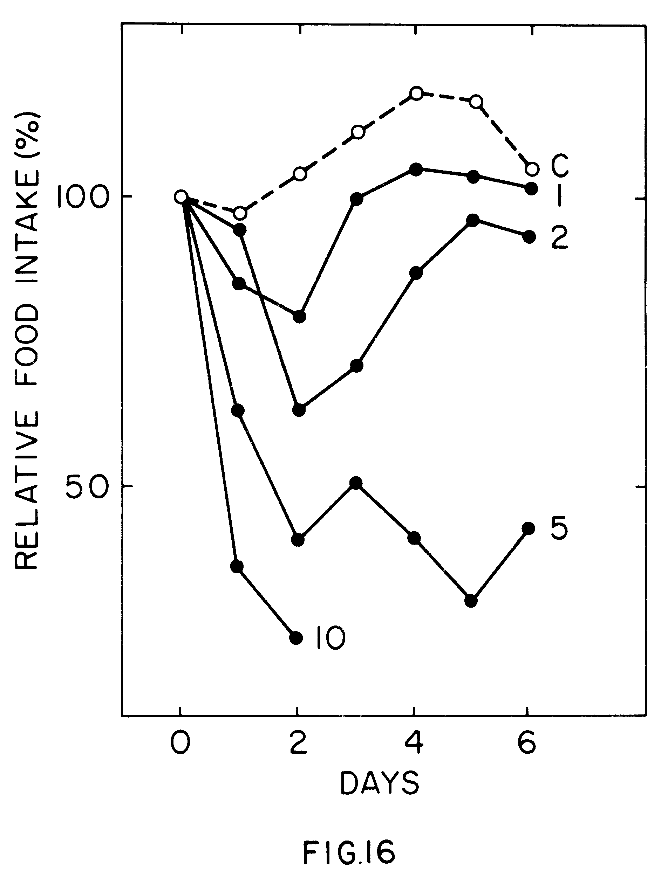

Figure 16 illustrates the effect of the macrophage mediator on food intake. Five groups of C3H/HeJ mice (six mice per group) received twice daily intraperitoneal injections (0.5 ml each) of the following solutions (C) RPMI 1640 containing 50 $\mu$g/ml endotoxin (control), (1) endotoxin activated macrophage culture medium (macrophage mediator), (2) macrophage mediator concentrated 2X, (3) macrophage mediator concentrated 5X, (4) macrophage mediator concentrated 10X. The average food intake of the animals in each group at Day 0 was taken as 100%. Metabolic cages (three animals per group) were utilized.

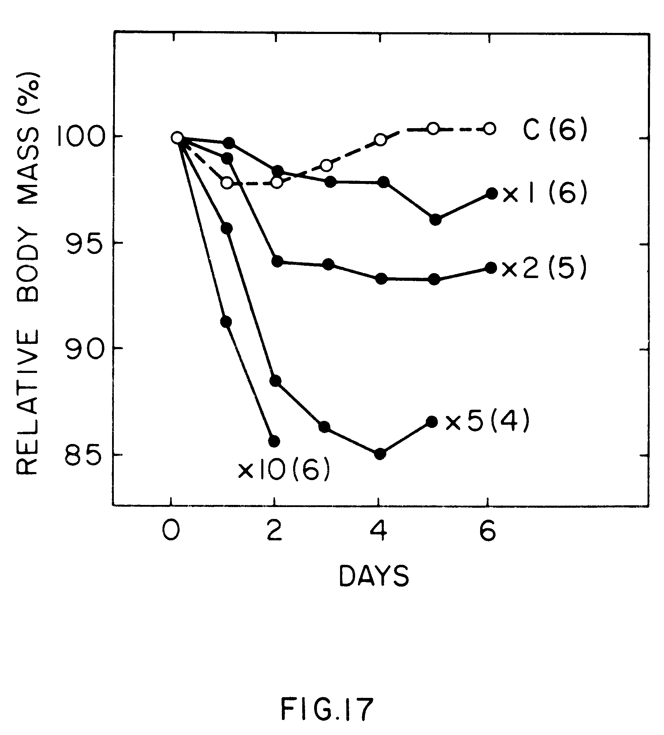

Figure 17 illustrates the relative change in body mass of animals corresponding to the experiment in Figure 16. The average of the body mass of the animals in each group at day 0 was taken as 100%.

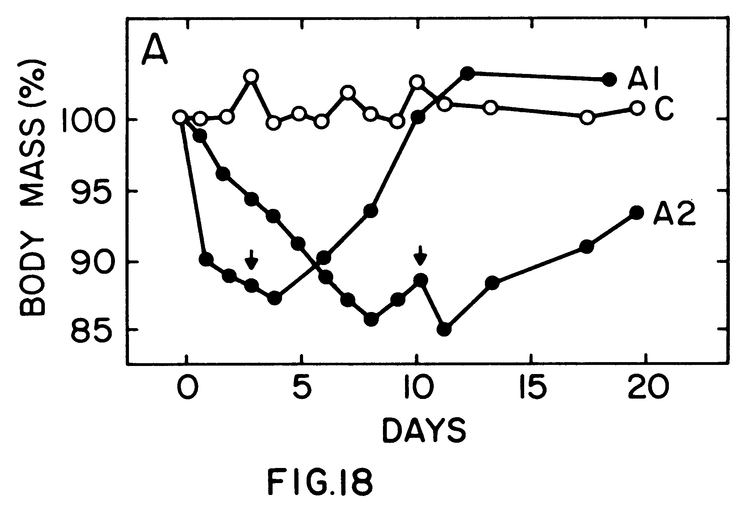

Figure 18 depicts the effect of discontinuation of the macrophage mediator on body mass. These groups of C3H/HeJ mice (five mice per group) received twice daily injections (0.5 ml each) of the following solutions (C) RPMI 1640 containing 50 $\mu$g/ml endotoxin (control), (A) 10X concentrated macrophage mediator (one mouse in this group died on Day 2). 5X concentrated macrophage mediator. The average body mass of the animals in each group at Day 0 was taken as 100%.

RESULTS

Mice receiving the mediator substance from endotoxin-activated macrophages exhibited a biphasic decrease in food intake which was dose dependent (Figure 16). Initially, all groups of mice receiving the mediator sharply decreased their food intake. This acute phase, lasting two days, was followed by the second phase in which mice would either increase their food intake to the level of controls (low dose) or continue to limit their food intake (high dose). This second phase was dependent on the quantity of macrophage mediator injected. The group which received the highest dose of mediator showed a dramatic decrease in food intake (60%), until they died on Day 3 after the initial dose. The water intake in all groups was comparable to food intake (data not shown).

The change in body mass of the animals in the preceeding experiment is shown in Figure 17. Body mass, like food intake, decreased in a dose-dependent biphasic fashion with respect to the mediator concentration. Mice receiving the smallest dose of mediator continued to lose weight despite normal food intake as compared to the control mice. Mice receiving higher doses lost up to 15% of their initial body mass. With the highest dose used, all six mice died after two days of treatment. Food intake was normal for the six mice in the control group which received a daily dose of 200 $\mu$g of endotoxin.

The cachectic effect observed in association with the macrophage mediator could be reversed by discontinuation of its administration (Figure 18). Two solutions of macrophage mediator similar to the two solutions with high concentrations used in the experiment of Figure 17 were injected into two groups of mice (three mice per group), but limited in two days with the highest dose (curve Al) or continued until ten days with the less concentrated solution (curve A2). Except for one mouse which did not survive in the former group, all other mice returned to the initial body weight.

To assure against possible bacterial contamination, blood was cultured from the animals injected and found to be sterile. Gross and micropathological surveys of animals receiving the macrophage mediator did not reveal any noteworthy findings.

Certain aspects of the above weight loss experiments were performed with the limited quantities of the modulator material, and while formal data were not collected and presented as above, it was observed by the authors that the modulator material exhibited an equivalent ability to cause weight loss in mice.

During the period of administration of the mediator and during which food intake was measured, the temperature of the animals was also measured and it was observed, remained constant and did not rise. The only change in temperature came within the last day prior to mortality, at which time the animals became moribund and expected temperature decreases were noted. The isolate cachectin when injected into C3H/HeJ mice also did not cause fever.

Based upon the results of the temperature measurements, it was concluded that, while Interleukin 1 affects the development of fever, such activity is not present with respect to either the modulator material or its precursor, the mediator substance.

EXAMPLE 9

A further activity that has been considered in the comparison between the mediator substance, its isolate cachectin, and Interleukin 1 is the activity of protein degradation. Like the promotion of fever, protein degradation has been identified in the literature as an activity exhibited by Interleukin 1. See Dinarello supra., Pages 71-72, and in particular, Subheading A. on Page 72. It therefore became desirable to determine whether the mediator substance and its isolate, the present modulator material, exhibited similar activities. Accordingly, muscle strips were incubated the mediator substance and the isolate, respectively, in like amounts to those wherein purified IL-1 had been administered. Thereafter, the muscle strips were observed, however, muscle proteolysis was not found. On this basis, it was determined that neither the mediator substance nor the modulator material took part in protein degradation in distinction to Interleukin 1.

EXAMPLE 10

Among the utilities to which the present modulator may be directed, it has been postulated that the modulator may be used to develop antibodies which may then be put to therapeutic uses, such as the inhibition or aversion of shock induced by certain bacterial infections. In an unpublished investigation conducted by Beutler, Milsark and Cerami, listed as item (6) under Related Publications, a specific polyclonal rabbit antiserum was developed against murine cachectin and BALB/c mice were passively immunized therewith both before, concurrently with and after administration of E. coli lipopolysaccharide (LPS). The procedures and results are reproduced below.

MATERIALS AND METHODS

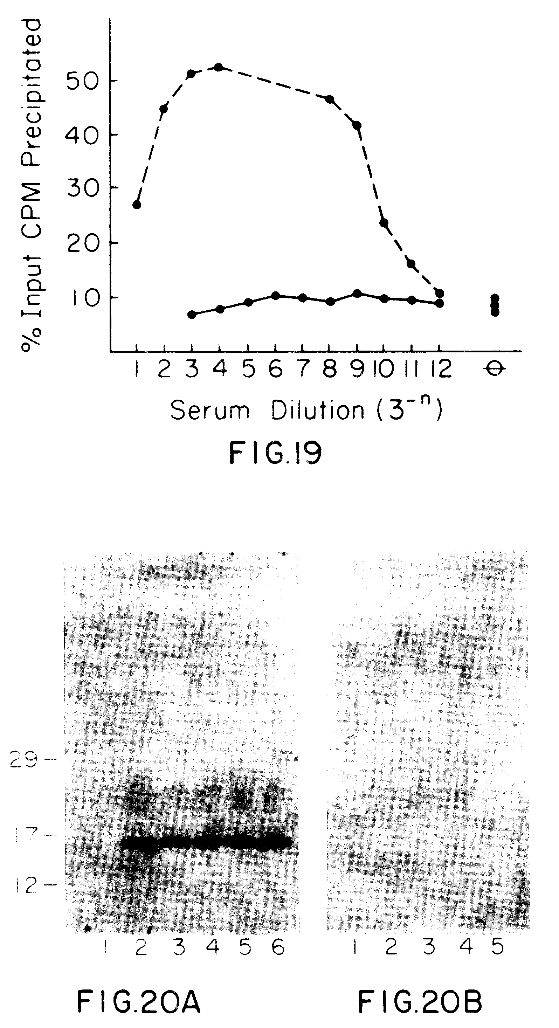

The modulator material cachectin was purified as previously described in Example 2. The purified protein was prepared for use in immunization by electrophoresis in an SDS-polyacrylamide slab gel. The gel was sliced after completion of electrophoresis, and approximately 5 $\mu$g of homogeneous protein (still contained within the gel slice) was emulsified in 1.0 ml of 0.05 M ammonium acetate solution and 1.0 ml of Freund's complete adjuvant. A New Zealand White female rabbit was injected at multiple subcutaneous sites with this material. Four additional injections were made at monthly intervals, using 2-5 $\mu$g of cachectin, prepared as above, using Freund's incomplete adjuvant. The rabbit was bled four days and six days after the final injection. Immune serum was tested for its ability to precipitate cachectin labelled with $^{125}$I by the iodogen method (Fig. 19). Approximately 50% of the tracer was precipitated using a 1:30,000 dilution of serum; pre-immune serum was non-reactive. The specificity of immune and pre-immune sera were analyzed by immunoblotting (Fig. 20). A single major species, corresponding to murine cachectin, was labelled when blot transfers of medium from RAW 264.7 cells previously incubated with LPS were exposed to the immune serum. Pre-immune serum showed no reactivity. Neither immune nor pre-immune serum contained antibodies reactive with LPS as assessed by the method of Neter, et al., J. IMMUNOL., 76:377 (1956), or by immunoblotting of LPS.

When immune serum was administered to male BALB/c mice by intraperitoneal injection one and one-half hours prior to intraperitoneal injection of 400 $\mu$g of LPS, a significant protective effect was demonstrated (Table V, below) compared to the mortality rate observed among control mice treated with pre-immune or non-immune sera. After administration of the LPS, the mice in all of the groups appeared ill, and exhibited a febrile response (data not shown). Thus, the immune serum did not abrogate the pyrogenic effect of LPS. A similar level of protection was observed when IgG prepared from the immune serum was administered to mice prior to injection of LPS. On the other hand, IgG prepared from non-immune serum did not protect the mice (data not shown).

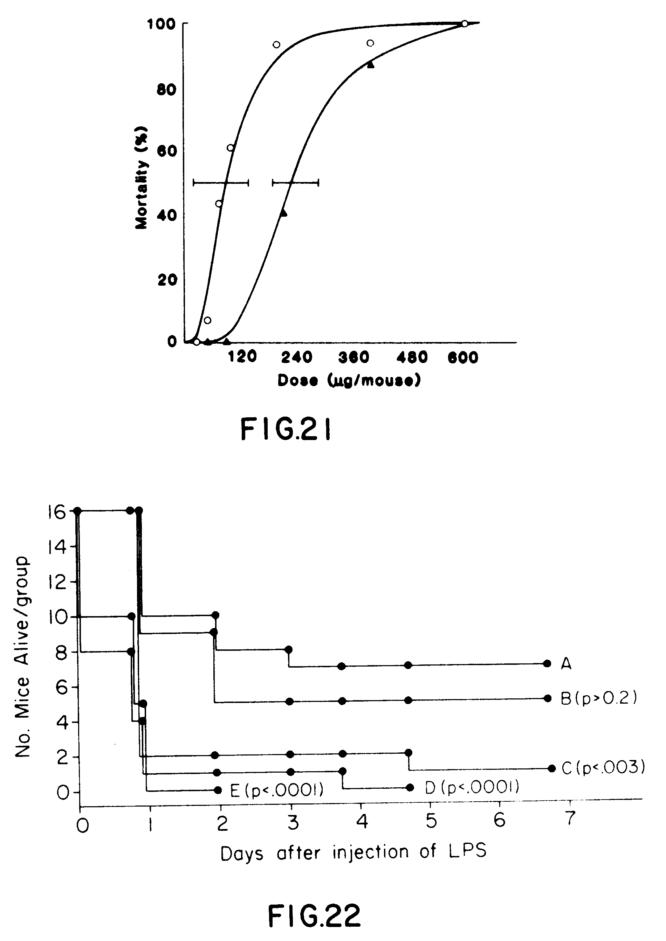

In order to assess the degree of protection provided by the immune serum, varying doses of LPS were administered to mice that had received either 50 $\mu$l of non-immune serum or 50 $\mu$l of immune serum five hours previously (Fig. 21). It is apparent that the $LD_{50}$ of LPS in animals treated with immune serum is significantly higher than the $LD_{50}$ for control mice treated with non-immune serum.

The time at which the antiserum is administered relative to the time of LPS administration, was found to be of crucial importance in producing a protective effect. Mice that were injected with immune serum three or six hours prior to administration of LPS fared significantly better than those passively immunized at the time of LPS injection or several hours after (Fig. 22). This finding suggests that endotoxin elicits cachectin production soon after its administration, and that cachectin mediates its lethal injury within a very short time. In rabbits, cachectin is produced within minutes following intravenous administration of LPS, and peak plasma levels are observed after two hours, with a rapid decline in levels occurring thereafter. Hence, in this model, the animal is exposed to high levels of the hormone only briefly; it is within this interval of time that effective antibody concentrations must be present if protection is to be achieved.

The data presented above give evidence for the role of cachectin in mediating the lethal effects of LPS. Cachectin is clearly only one of the mediators responsible for the numerous pathological effects evoked by LPS, since the passively immunized mice become febrile, and continue to appear ill and distressed. It is possible, for example, that cachectin acts in concert with other mediators (e.g., Interleukin 1, interferons, and lymphotoxin) in order to elicit the lethal effect of LPS.

It is important to note that mice are relatively resistant to the effects of LPS when compared to most other mammals; rabbits, for example, are approximately 1000-fold more sensitive. In LPS-sensitive species, cachectin may play a more prominent role as a mediator of shock. Immunization against cachectin might then be expected to yield a higher level of protection.

The potential utility of passive immunization with antisera to cachectin in animals with shock induced by septicemia (or possibly other causes) needs further exploration. An obvious corollary is the possibility that agents when affect the synthesis or binding of cachectin to its receptor might be of utility in this setting without compromising the host's immune system.

The following figure lengends offer further detail as to the procedures employed and the depiction of the results thereof.

FIGURE LEGENDS

Figure 19 graphically depicts the immunoprecipitation of $^{125}$I-labelled murine cachectin. Immune serum (solid circles) was prepared as described in the text. Pre-immune serum was obtained from the same animal. Immunoprecipitation reactions were carrried out in conical polypropylene tubes. Serum samples wer diluted to yield the final concentrations indicated, using Dulbecco's phosphate buffered saline containing 1% RIA-grade bovine Serum albumin (Sigma Chemical Co., St. Louis, Missouri) (PBS/BSA). 0; no serum added. 2 $\mu$l (approximately 0.2 ng; 1.4 x 10$^4$ CPM) of labelled cachectin solution, prepared as previously described, was mixed with 20 $\mu$l of diluted serum. Solutions were incubated at 4°C for five hours. At that time, 10 $\mu$l of a 1:5 suspension of washed S. aureus bacterial adsorbant (Miles Laboratories, Inc., Elkhart, Indiana) in distilled water was added to each tube. After thirty minutes, 1.0 ml of PBS/BSA was added to each tube. Samples were quickly mixed, and the immunoprecipitates were sedimented using a Beckman microfuge. Supernatants were aspirated, and the pellets counted for radioactivity using a Packard Autogamma Scintillation Spectrometer, Model 578. Results are expressed as precent of imput CPM precipitated. Non-immune sera from other rabbits (data not shown) were also incapable of precipitating labelled cachectin.

Figure 20 is an autoradiograph of a Western blot of crude RAW 264.7 cell conditioned medium, and of LPS. Lane 1 (a and b): 50 $\mu$g E. coli strain 0127:B8 LPS (Difco; Detroit, Michigan). Lanes 2-6 (a) and 2-5 (b): 25 $\mu$l of LPS-stimulated RAW 264.7 cell (mouse macrophage) conditioned medium (containing approximately 40 $\mu$g total protein), prepared and concentrated 50-fold as previously described in Example 1. Samples were applied to a 10-15% SDS-polyacrylamide gradient gel. After completion of electophoresis, protein was transferred to nitrocellulose paper (Schleicher and Schuell, Inc., Keene, New Hampshire) using a electroblotting apparatus (Bio-rad Laboratories, Richmond, California). Immune (a), and pre-immune (b) sera were applied to the nitrocellulose blot at dilutions of 1:300, and reactive bands were identified with $^{125}$I-labelled protein A (New England Nuclear Corp., Boston Massachusetts). The band visible in (a) corresponds precisely in molecular weight to murine cachectin, when the purified radioiodinated protein is electrophoresed and transferred in parallel as a marker. No reactive proteins are apparent when pre-immune serum is applied to the blot.

Figure 21 is a graphical plot of the estimation of the endotox in $LD_{50}$ in mice with immune and non-immune sera. Male BALB/c mice (19-21 g) were randomized to eleven groups, each containing fifteen to seventeen members. In four groups (triangles), mice were pretreated by intraperitoneal injection of 200 $\mu$l of immune serum diluted 1:4 with sterile isotonic saline; in the remaining seven groups (open circles), mice were pretreated with the same volume of 1:4 diluted non-immune serum. After five hours, mice were given intraperitoneal injections of varying quantities of E. coli strain 0127:B8 LPS, dissolved in sterile saline. Survival was followed in all groups of forty-eight hours after LPS injection. Curves wer fitted to the data, and computation of $LD_{50}$ was accomplished as described by Bliss, Q., J. PHARMACOL., 11:192 (1938), and by Litchfield, et al., J. PHARMACOL. EXP. THER., 96:99 (1949). Horizontal bars indicated that 95% confidence limits of $LD_{50}$ determinations (91 $\mu$g for mice treated with non-immune serum, and 227 $\mu$g for mice treated with immune serum). The administration of larger volumes of immune serum to mice (up to 200 $\mu$l) does not further improve survival (data not shown).

Figure 22 depicts a Kaplan-Meier survival plot following LPS treatment. 80 male BALB/c mice (19-21 g) were randomized to five groups of sixteen members each. Mice were protected by intraperitoneal injection of 200 $\mu$l of anti-cachectin serum either six or three hours before, concurrent with, or three to six hours following intraperitoneal injection of 400 $\mu$g of E. coli strain 0127:B8 LPS (Difco; Detroit, Michigan). Censored determinations of survival were made at the indicated time points following LPS injection. Survival was optimal when mice were pretreated six hours prior to LPS administration (A). A significant decrease in survival, with respect to these values, was noted when mice were given anti-cachectin serum at the time of LPS administration (C), three hours after LPS administration (D), or six hours after LPS administration (E). Tests of the null hypothesis that the time-related probability of survival in each group was equal to that in the group protected six hours prior to LPS administration were performed using the Gehan method (See Gehan, BIOMETRIKA, 52:203 (1965); p values (single tail normal distribution) are indicated for each curve.

```
serum volume (  1)¹:        10        50        200

            immune          3/14      6/14      7/14

     non-immune             0/14      1/14      0/14

                            p<0.1²    p<0.05    p<0.001
```

Table V. [1]Female BALB/c mice (20-24 g) were randomized to six groups and pretreated by intraperitoneal injection of serum from immune or non-immune rabbits one and one-half hours prior to intraperitoneal injection of 400 $\mu$g E. coli strain 0127:B8 LPS. Serum samples were diluted with sterile isotonic saline, and injected in a final volume of 0.2 ml per mouse. LPS was also diluted in sterile saline, and injected in a volume of 0.2 ml. Mortality was recorded daily, and the experiment was considered complete when no deaths were observed in any group for a period of three days. The figures above reflect the number of survivors at seven days post LPS injection.

[2]double-tailed t distribution.

From the above example, it is apparent that the present modulator material indeed possesses the therapeutic capabilities that have been theorized to be attributable to it. Thus, to the extent that the modulator material or its binding partner may be appropriately administered, abatement or alleviation of the adverse conditions causally related to the undesirable degree to which the cellular and systemic condition of the patient resides in either the anabolic or the catabolic state can be achieved. The modulator therefore represents an effective therapeutic tool the full potential of which has not yet been realized.

**Claims**
**Claims for the following Contracting States : BE, CH, DE, FR, GB, IT, LI, LU, NL, SE**

1.  A pharmaceutical composition for the treatment of cachexia and shock in humans, comprising:
    (a) a pharmaceutically effective amount of an anticachectin antibody; and
    (b) a pharmaceutically acceptable carrier.

2.  The pharmaceutical composition of claim 1, wherein said cachectin is derivable from animal macrophage cells that have been incubated with a stimulator material that accompanies an invasive stimulus.

3.  The pharmaceutical composition of claim 1 or 2, wherein said cachectin is derived from cells which are produced by clonal replication.

**Claims for the following Contracting State : AT**

1.  Method for the preparation of a pharmaceutical composition for the treatment of cachexia and shock in humans which comprises combining a pharmaceutically effective amount of an anti-cachectin antibody and a pharmaceutically acceptable carrier.

2.  The method of claim 1, wherein said cachectin is derivable from animal macrophage cells that have been incubated with a stimulator material that accompanies an invasive stimulus.

3.  The method of claim 1 or 2, wherein said cachectin is derived from cells which are produced by clonal replication.

**Patentansprüche**
**Patentansprüche für folgende Vertragsstaaten : BE, CH, DE, FR, GB, IT, LI, LU, NL, SE**

1.  Arzneimittel zur Behandlung einer Kachexie und eines Schockzustandes bei Menschen, umfassend:
    (a) eine pharmazeutisch wirksame Menge eines anti-Cachectin-Antikörpers, und
    (b) einen pharmazeutisch verträglichen Träger.

2.  Arzneimittel nach Anspruch 1, wobei das Cachectin erhältlich ist aus tierischen Makrophagenzellen, die mit einem Reizmittel inkubiert wurden, das einen invasiven Reiz begleitet.

3.  Arzneimittel nach Anspruch 1 oder 2, wobei das Cachectin von Zellen stammt, die durch clonale Vermehrung hergestellt werden.

**Patentansprüche für folgenden Vertragsstaat : AT**

1.  Verfahren zur Herstellung eines Arzneimittels zur Behandlung einer Kachexie und eines Schockzustandes bei Menschen, umfassend die Kombination einer pharmazeutisch wirksamen Menge eines anti-Cachectin-Antikörpers und eines pharmazeutisch verträglichen Trägers.

2.  Verfahren nach Anspruch 1, wobei das Cachectin aus tierischen Makrophagenzellen erhältlich ist, die mit einem Reizmittel inkubiert wurden, das einen invasiven Reiz begleitet.

3.  Verfahren nach Anspruch 1 oder 2, wobei das Cachectin von Zellen stammt, die durch clonale Vermehrung hergestellt werden.

**Revendications**
**Revendications pour les Etats contractants suivants : BE, CH, DE, FR, GB, IT, LI, LU, NL, SE**

1. Composition pharmaceutique pour le traitement de la cachexie et du choc chez l'homme, comprenant:
   (a) une quantité pharmaceutiquement efficace d'un anticorps anti-cachexine; et
   (b) un support pharmaceutiquement acceptable.

2. Composition pharmaceutique de la revendication 1, dans laquelle ladite cachexine peut être obtenue à partir de cellules macrophages animales que l'on a fait incuber avec une matière stimulante qui accompagne un stimulus invasif.

3. Composition pharmaceutique de la revendication 1 ou 2, dans laquelle ladite cachexine est dérivée de cellules qui sont produites par réplication clonale.

**Revendications pour l'Etat contractant suivant : AT**

1. Procédé de préparation d'une composition pharmaceutique pour le traitement de la cachexie et du choc chez l'homme, dans lequel on combine une quantité pharmaceutiquement efficace d'un anticorps anticachexine et un support pharmaceutiquement acceptable.

2. Procédé de la revendication 1, dans lequel ladite cachexine peut être obtenue à partir de cellules macrophages animales que l'on a fait incuber avec une matière stimulante qui accompagne un stimulus invasif.

3. Procédé de la revendication 1 ou 2, dans lequel ladite cachexine est dérivée de cellules qui sont produites par réplication clonale.

FIG.I

FIG.2

FIG.3

FIG.4

FIG.5A

FIG.5B

% Input Counts Bound

Incubation Time (hours)

−Competition

+25,000 u/ml Competition

FIG.6A

FIG.6B

EP 0 212 489 B1

FIG.7

FIG.8

FIG.9

FIG.10

% Maximal Suppressive Effect vs Antibody dilution ($2^n$) in reaction mix

No Antibody Added

EP 0 212 489 B1

FIG.IIA

FIG.IIB

FIG.12

FIG.13

FIG.14

## FIG.I5A    FIG.I5B

**Hours**
0  6  12  24

Clone 47

● GPD
◆ Clone 1

Maximum expression (%)

Time (hours)

EP 0 212 489 B1

FIG.16

FIG.17

FIG.18

FIG.19

FIG.20A          FIG.20B

FIG.21

FIG.22

```
LEU ARG SER SER SER GLN ASN SER SER ASP LYS PRO VAL ALA
HIS VAL VAL ALA ASN HIS GLN VAL GLU GLU GLN LEU GLU TRP
LEU SER GLN ARG ALA ASN ALA LEU LEU ALA ASN GLY MET ASP
LEU LYS ASP ASN GLN LEU VAL VAL PRO ALA ASP GLY LEU TYR
LEU VAL TYR SER GLN VAL LEU PHE LYS GLY GLN GLY CYS PRO
ASP TYR VAL LEU LEU THR HIS THR VAL SER ARG PHE ALA ILE
SER TYR GLN GLU LYS VAL ASN LEU LEU SER ALA VAL LYS SER
PRO CYS PRO LYS ASP THR PRO GLU GLY ALA GLU LEU LYS PRO
TRP TYR GLU PRO ILE TYR LEU GLY GLY VAL PHE GLN LEU GLU
LYS GLY ASP GLN LEU SER ALA GLU VAL ASN LEU PRO LYS TYR
LEU ASP PHE ALA GLU SER GLY GLN VAL TYR PHE ARG VAL ILE
ALA LEU
```

# FIG.23

EP 0 212 489 B1